# EUROPEAN PATENT APPLICATION

(11) **EP 2 886 647 A1**
(43) Date of publication of application: **24.06.2015**
(21) Application number: 13198756.2
(22) Date of filing: 20.12.2013
(51) Int. Cl.: C12N 9/16, C12N 15/52

(54) **Polypeptides with diterpene synthase activity**

(71) Applicant: Technical University of Denmark, 2800 Kgs. Lyngby (DK); University of Copenhagen, 1165 Copenhagen K (DK); THE UNIVERSITY OF BRITISH COLUMBIA, Vancouver, British Columbia V6T 1Z3 (CA)
(72) Inventor: Hamberger, Björn, 2770 Kastrup (DK); Pateraki, Eirini, 2450 Copenhagen SV (DK); Lindberg Møller, Birger, 2700 Brønshøj (DK); Nørholm, Morten, 3400 Hillerød (DK); Thrane Nielsen, Morten, 2200 Copenhagen N (DK); Andersen-Ranberg, Johan, 2200 Copenhagen N (DK); Bohlmann, Carl Jörg, Vancouver, British Columbia V6S 1T4 (CA); Zerbe, Philip, North Vancouver, British Columbia V7G1C3 (CA)
(74) Representative: Høiberg A/S

(57) **Abstract**

The present invention relates to polypeptides with diterpene synthase activity. The invention further relates to polynucleotides encoding such polypeptides. Also provided are vectors for expression of the polypeptides and host cells expressing the polypeptides.

## Description

### Field of invention

The present invention relates to polypeptides with diterpene synthase activity. The invention further relates to polynucleotides encoding such polypeptides. Also provided are vectors for expression of the polypeptides and host cells expressing the polypeptides.

### Background of invention

Manoyl oxide is a compound which exhibits a number of important properties like antibacterial, anticancer and anti-inflammatory activities. Manoyl oxide has so far only been detected as a side product or artefact of some reactions, and was present in a racemic mixture. Manoyl oxide derivatives also present numerous attractive properties. The (13R)-manoyl oxide epimer is a putative precursor of forskolin, a labdane diterpenoid found in the root of *Coleus forskohlii* (family *Lamiaceae*) which has received much attention for its broad range of pharmacological activities. However, despite extensive studies focusing on its medicinal properties, the biosynthesis of forskolin has not yet been conclusively elucidated.

*Coleus forskohlii* (synonym: *Plectranthus barbatus*) is a perennial medicinal shrub of the mint family (Lamiaceae) indigenous to the subtropical and temperate climate zones of India and south-east Asia. The plant has been used since ancient times in Hindu and Ayurvedic traditional medicine for treating a broad range of human health disorders. The main active compound of C. *forskohlii* is forskolin, a heterocyclic labdane type diterpene found in the roots of the plant. The diverse pharmaceutical known and potential applications for forskolin extend from alleviation of glaucoma, anti-HIV or antitumor activities to treatment of hypertension and heart failure. The efficacy of forskolin relies on activation of the adenylate cyclase enzyme leading to a marked increase of the intracellular level of cAMP (3'-5'-cyclic adenosine monophosphate) in mammalian *in vitro* and *in vivo* systems. The semi-synthetic forskolin derivative NKH477 has been approved for commercial use in Japan for treatment of cardiac surgery complications, heart failure, and cerebral vasospasm, while a forskolin eye drop solution was recently approved as an effective treatment for glaucoma.

The chemical complexity of *C*. *forskohlii* has been well studied and a total of 68 different diterpenoids have been isolated and identified from different tissues of the plant, of which 25 belong to the class of abietanes and 43 to the class of labdanes. While the tricyclic abietane diterpenes have been reported to accumulate predominantly in the aerial parts, labdane diterpenoids with a bicyclic decalin core were detected primarily in the roots. Forskolin is a representative of an unusual series of tricyclic (8,13)-epoxy-labdanes, characteristic for this plant.

Manoyl-oxide has to this date only been detected as experimental artefact (Zerbe et al., 2012; Günnewich et al., 2013). Enzymatic conversion leading to production of manoyl oxide has at present never been reported. Thus polypeptides capable of producing manoyl oxide are needed.

### Summary of invention

The invention relates to polypeptides having a diterpene synthase (diTPS) activity. The invention further relates to polynucleotides encoding such polypeptides and to vectors comprising such polynucleotides. The invention finally relates to a host cell comprising such vectors and/or such polynucleotides. The polypeptides of the invention are relevant for catalysing enzymatic synthesis of manoyl oxide.

In one aspect, the invention relates to polypeptides having a diterpene synthase activity and comprising:
i) an amino acid sequence selected from the group consisting of SEQ ID NO: 1 [CfTPS2], SEQ ID NO: 2 [CfTPS3], SEQ ID NO: 3 [CfTPS4] and SEQ ID NO: 4 [EpTPS8];
ii) a biologically active sequence variant of said polypeptide, wherein the sequence variant has at least 75% sequence identity to said SEQ ID NO: 1 [CfTPS2], SEQ ID NO: 2 [CfTPS3], SEQ ID NO: 3 [CfTPS4] or SEQ ID NO: 4 [EpTPS8];
iii) a biologically active fragment of at least at least 50 contiguous amino acids of any of i) through ii), said fragment having at least 75% sequence identity to SEQ ID NO: 1 [CfTPS2], SEQ ID NO: 2 [CfTPS3], SEQ ID NO: 3 [CfTPS4] or SEQ ID NO: 4 [EpTPS8] in a range of overlap of at least 50 amino acids,
   wherein the biological activity is diterpene synthase activity.

In a further aspect, the invention relates to a polynucleotide encoding a polypeptide of the invention.

In another aspect, the invention relates to a vector comprising at least one polynucleotide of the invention.

In yet another aspect, the invention relates to a cell comprising a polynucleotide of the invention and/or a vector of the invention.

### Description of Drawings

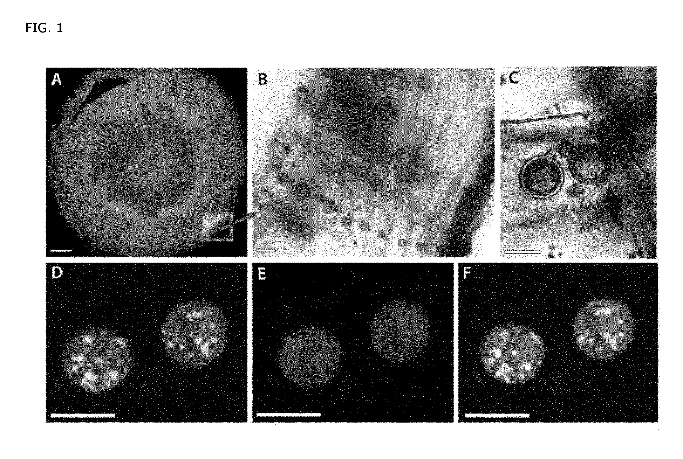

**Figure 1****:** Localization of oil bodies within the root cork of C. *forskohlii.* (A) Cross section of entire root with thick fissured cork. Lower right inset: the location of cork cells. (B) Rows of cork cells of different intensity, each with one prominent oil body. (C-F) Confocal imaging of Nile Red labeled oil bodies. (C) Cell with two oil bodies (same motif as D-F) seen in transmission channel. Discrimination between neutral lipids seen as bright deposits (D) and polar lipids seen in the magenta spectrum (E), also shown as overlay (F). The bar represents 200 µm (A) and 10 µm (B-F).

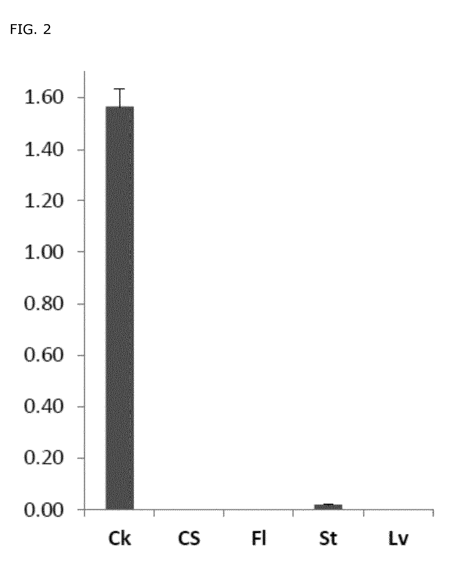

**Figure 2****:** Forskolin content (mg g⁻¹DW) as determined by HPLC-ELSD analysis from different tissues of C. *forskohlii.* (Ck), root cork; (CS), root stele and cortex; (FI), flowers; (St) stems and (Lv) leaves. Data are the mean ± SE of three independent biological replicates. The Y axis shows the forskolin content (mg/g dry weight).

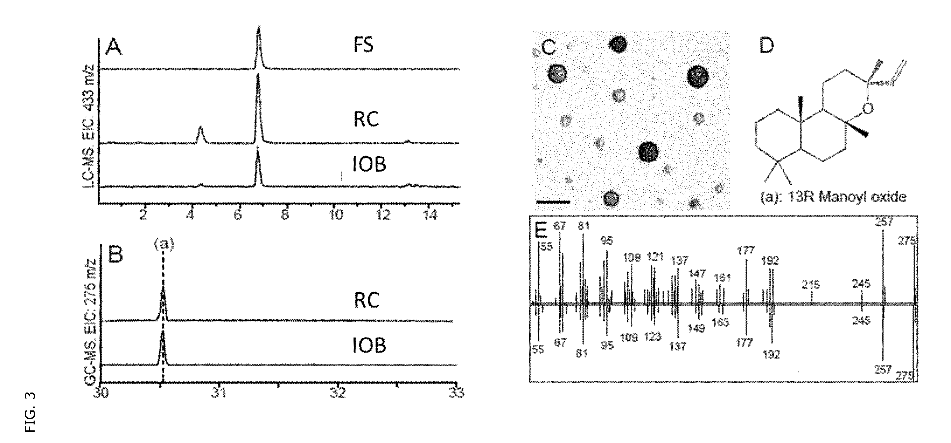

**Figure 3****:** Selected diterpenes detected in *C*. *forskohlii* oil bodies. FS: forskolin standard; RC: root cork; IOL: isolated oil bodies. (A) LC-MS analysis of forskolin (410 [forskolin]+23 [Na+]) in isolated oil bodies and in root cork tissue from C. *forskohlii.* The X axis shows the time in minutes. (B) GC-MS analysis of manoyl oxide in isolated oil bodies and in root cork tissue from *C*. *forskohlii.* The X axis shows the time in minutes. (C) Bright field microscope image of isolated C. *forskohlii* oil bodies. The bar represents 5 µm. (D) Molecular structure of (13R)-manoyl oxide. (E) Mass spectrum obtained from manoyl oxide identified in root cork tissue (top) and reference spectrum (bottom) from Wiley mass spectrum database.

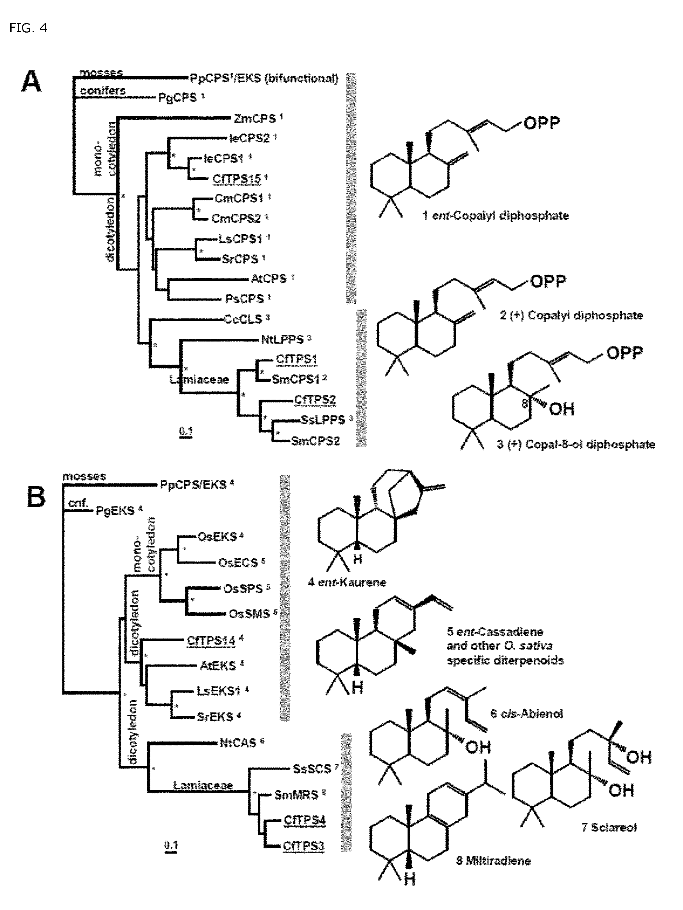

**Figure 4****:** Phylogenetic classification of *C*. *forskohlii* diterpene synthases with known class II (A) and class I (B) sequences. The phylograms are rooted with the bifunctional *ent*-copalyl diphosphate synthase/*ent*-kaurene synthase from the moss *Physcomitrella patens*. Asterisks indicate nodes supported by >80% bootstrap confidence and the scale bar indicates 0.1 amino acid changes. The numbers indicated at each enzyme are referred to the enzymatic products, the structures of which are given on the right.

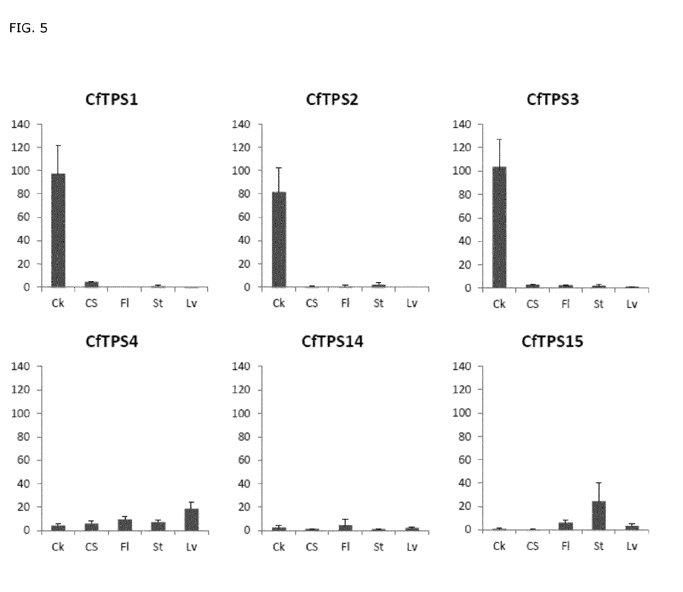

**Figure 5****:** Relative expression of *CfTPS* genes in *C*. *forskohlii* tissues. (Ck), root cork; (CS), root stele and cortex; (FI), flowers; (St), stems and (Lv), leaves. Transcript abundance of *CfTPS* genes expressed in arbitrary units was measured by qPCR using the translation initiation factor (TIF4a) for normalization. Each value represents the average of three biological replicates, each of which was performed in at least three technical replicates.

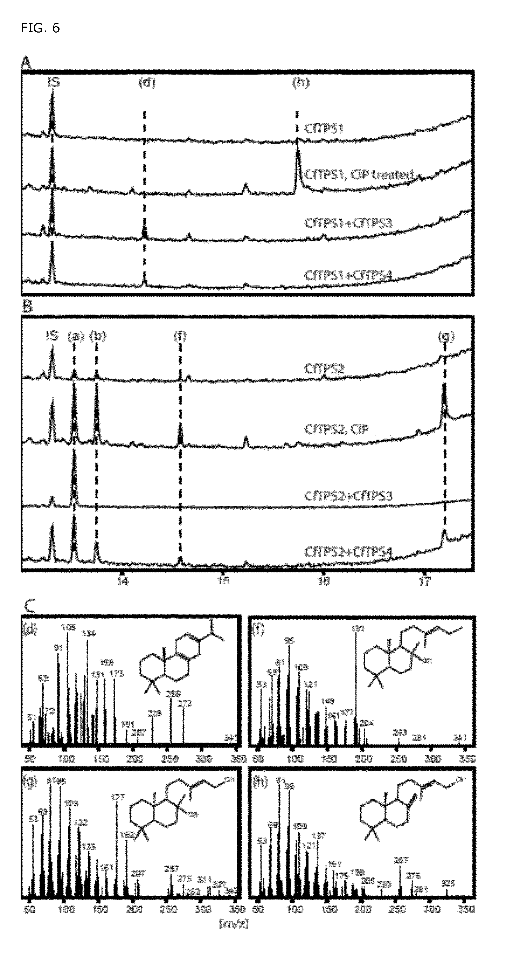

**Figure 6**: GC-MS analysis of *in vitro* assays with *C*. *forskohlii* diTPS. IS, internal standard (1ppm 1-eicosene). (A) *In vitro* assays with CfTPS2 alone and coupled assays with CfTPS2 and CfTPS3 and CfTPS4. Extracts of CfTPS2 assays were treated with calf intestinal alkaline phosphatase (CIP). The X axis shows the retention time (minutes). (B) *In vitro* assays with CfTPS1 and coupled with CfTPS3 and CfTPS4. Extracts of CfTPS1 were treated with CIP. The X axis shows the retention time (minutes). (a), (13R)-manoyl oxide; (b), (13S)-manoyl oxide; (g), labd-13-en-8,15-diol and (f), labden-8-ol; (d), miltiradiene and (h), copal-15-ol. (C) Mass spectra of compounds identified from assays. Structures tentatively identified as described in Materials and Methods.

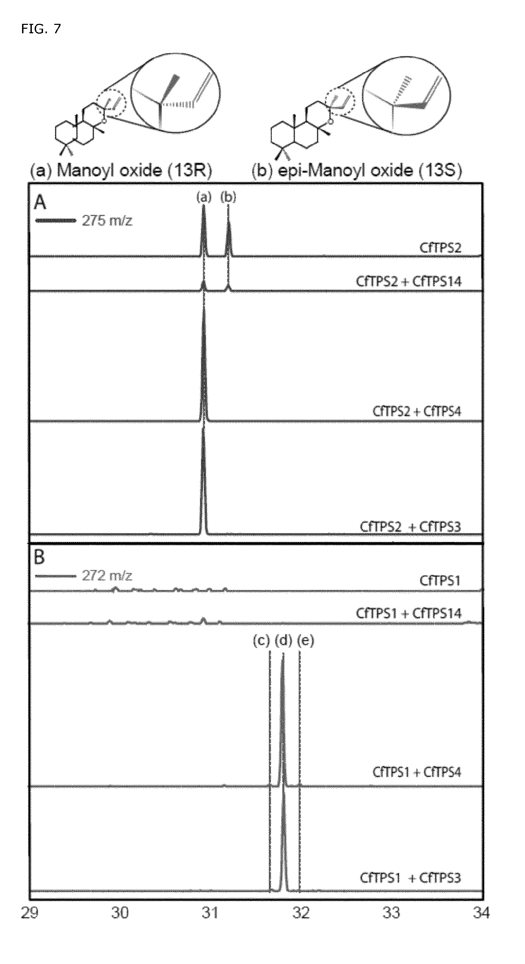

**Figure 7****:** GC-MS analysis of hexane extracts from *N. benthamiana* transiently expressing C. *forskohlii* diTPS. (A) Extracted ion chromatogram of EIC: 275 m/z. (a), (13R)-manoyl oxide and (b), (13S)-manoyl oxide. (B) Extracted ion chromatogram of EIC: 272 m/z. (c), dehydroabietadiene; (d), miltiradiene and trace amount of (e), abietadiene.

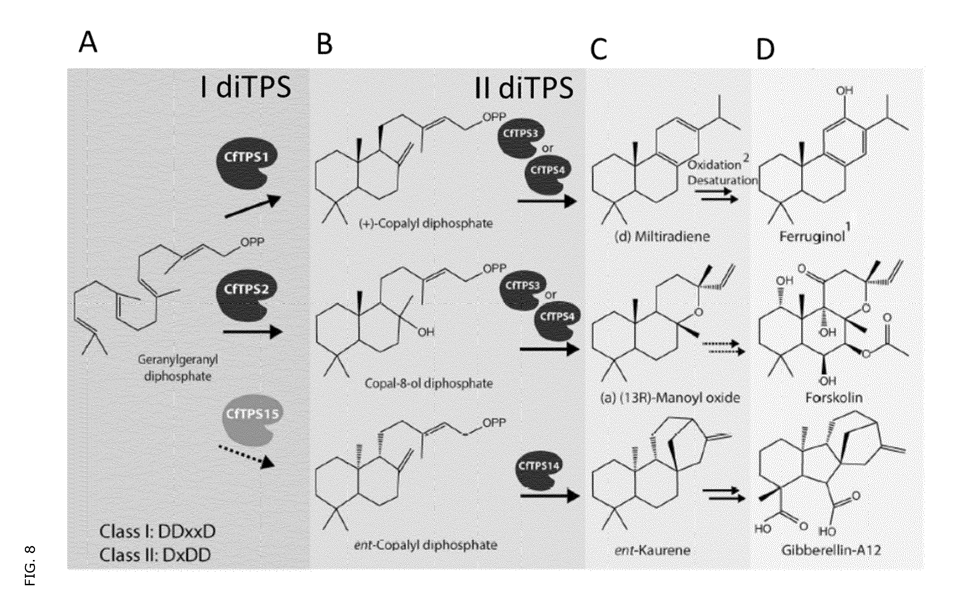

**Figure 8****:** Scheme of the biosynthetic routes from GGPP to specialized and general diterpenoids of the abietane, labdane and *ent*-kaurene class. Dashed arrows indicate reactions without experimental evidence in *C*. *forskohlii.* ¹Detection of (+)-ferruginol in *C*. *forskohlii* was reported earlier (Kelecom, 1983); ²CYP76AH1 from the close relative *Salvia miltiorrhiza* was shown to convert miltiradiene to ferruginol (Guo et al., 2013). A: universal precursor; B: diphosphate intermediates; C: diterpene backbone; D: representative functional diterpenoids.

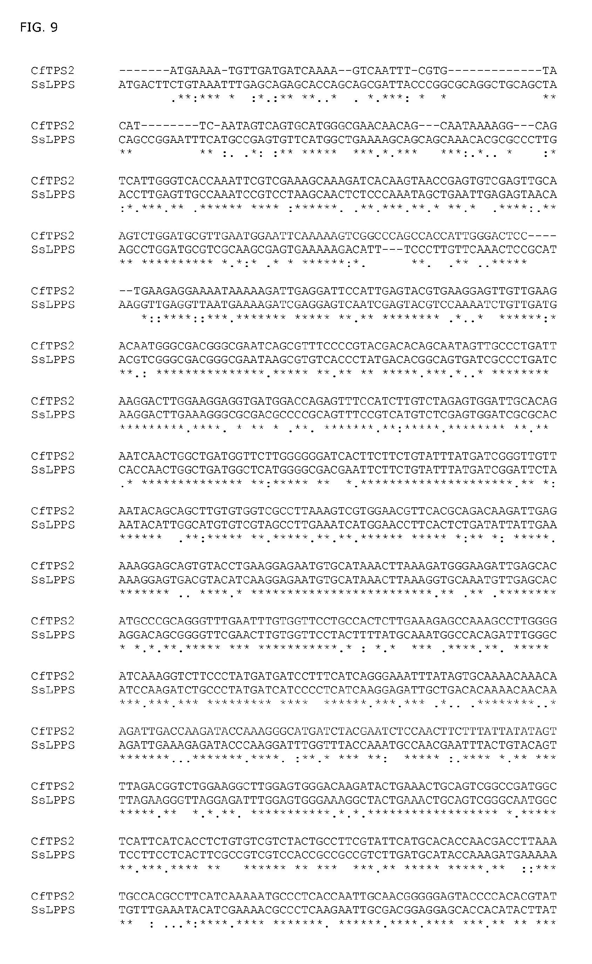

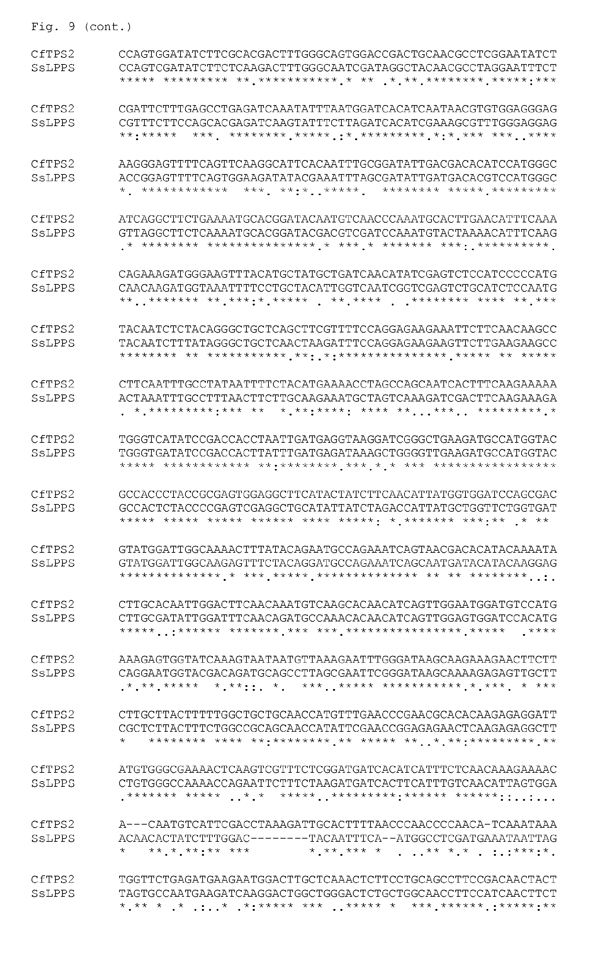

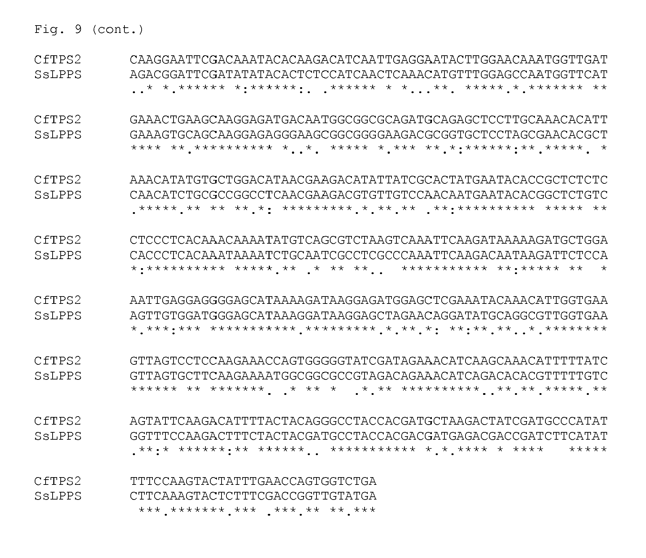

**Figure 9****:** Clustal alignment of the class II diTPS. The amino acid sequences of CfTPS2 and SsLPPS were aligned using the Clustal omega from the EMBL-EBI (http://www.ebi.ac.uk/Tools/msa/clustalo/help/).

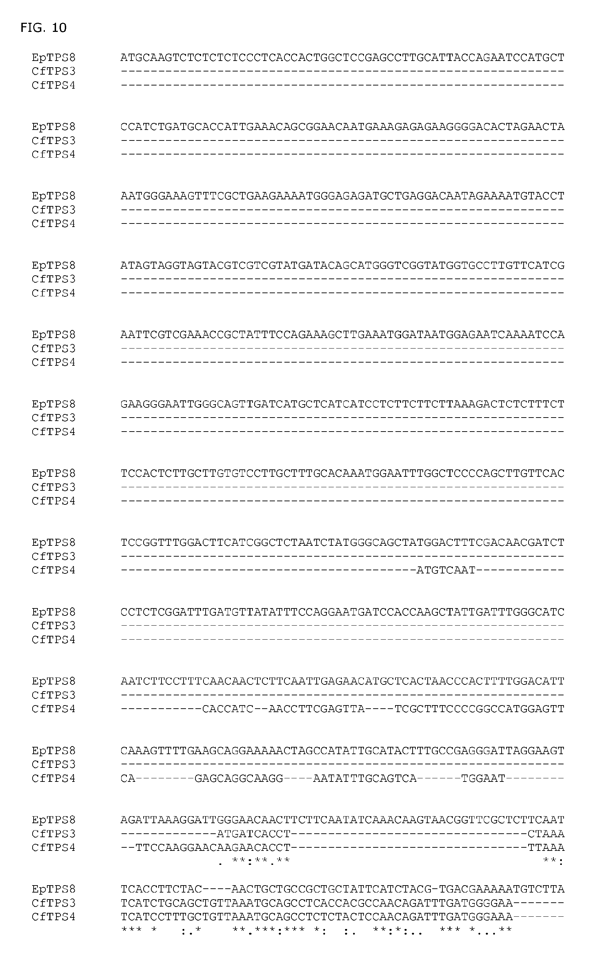

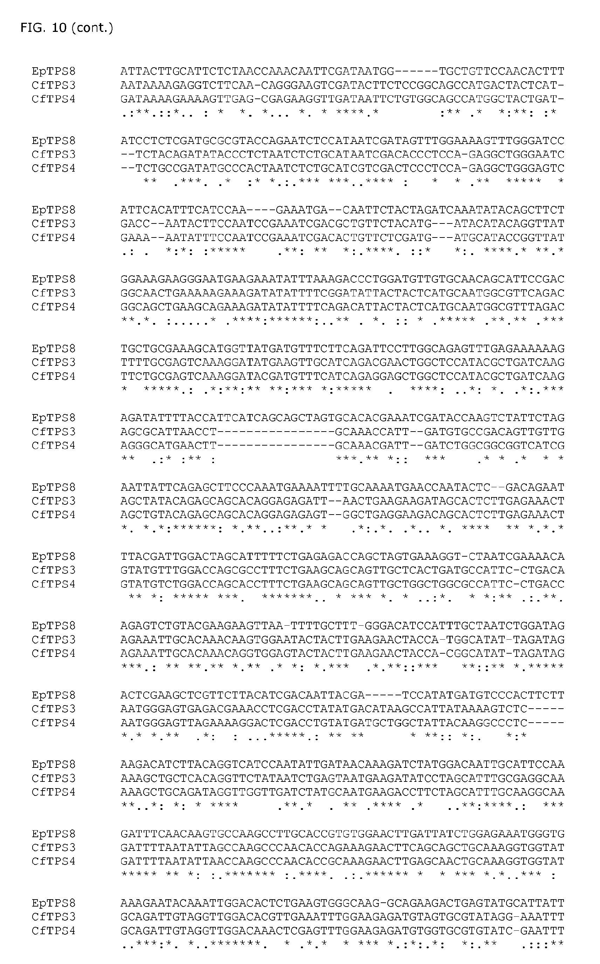

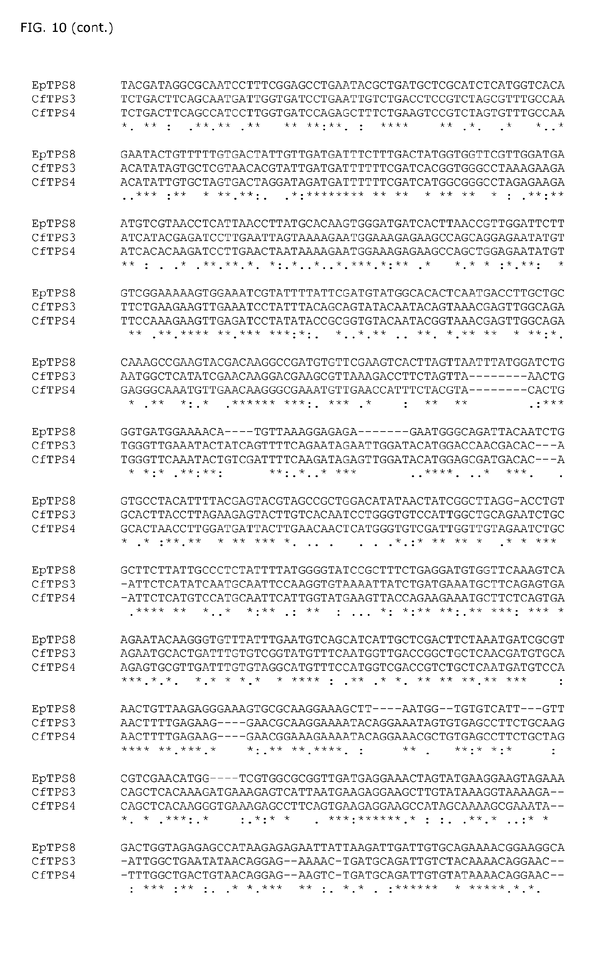

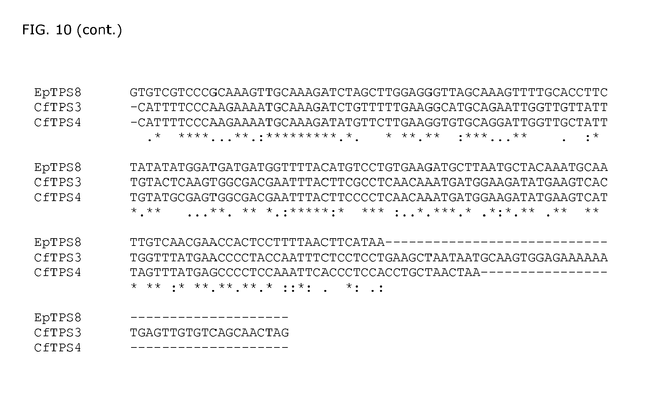

**Figure 10****:** Clustal alignment of the class I diTPS. The amino acid sequences of CfTPS3, CfTPS4 and EpTPS8 were aligned using the Clustal omega from the EMBL-EBI (http://www.ebi.ac.uk/Tools/msa/clustalo/help/).

### Detailed description of the invention

The invention relates to polypeptides having a diterpene synthase (diTPS) activity. The invention further relates to polynucleotides encoding such polypeptides and to vectors comprising such polynucleotides. The invention finally relates to a host cell comprising such vectors and/or such polynucleotides. The polypeptides of the invention are relevant for catalysing enzymatic synthesis of manoyl oxide.

In one aspect, the invention relates to polypeptides having a diterpene synthase activity and comprising:
i) an amino acid sequence selected from the group consisting of SEQ ID NO: 1 [CfTPS2], SEQ ID NO: 2 [CfTPS3], SEQ ID NO: 3 [CfTPS4] and SEQ ID NO: 4 [EpTPS8];
ii) a biologically active sequence variant of said polypeptide, wherein the sequence variant has at least 75% sequence identity to said SEQ ID NO: 1 [CfTPS2], SEQ ID NO: 2 [CfTPS3], SEQ ID NO: 3 [CfTPS4] or SEQ ID NO: 4 [EpTPS8];
iii) a biologically active fragment of at least at least 50 contiguous amino acids of any of i) through ii), said fragment having at least 75% sequence identity to SEQ ID NO: 1 [CfTPS2], SEQ ID NO: 2 [CfTPS3], SEQ ID NO: 3 [CfTPS4] or SEQ ID NO: 4 [EpTPS8] in a range of overlap of at least 50 amino acids,
   wherein the biological activity is diterpene synthase activity.

In a further aspect, the invention relates to a polynucleotide encoding a polypeptide of the invention.

In another aspect, the invention relates to a vector comprising at least one polynucleotide of the invention.

In yet another aspect, the invention relates to a cell comprising a polynucleotide of the invention and/or a vector of the invention.

### Definitions

Amino acid: Any synthetic or naturally occurring amino carboxylic acid, including any amino acid occurring in peptides and polypeptides including proteins and enzymes synthesized in vivo thus including modifications of the amino acids. The term amino acid is herein used synonymously with the term "amino acid residue" which is meant to encompass amino acids as stated which have been reacted with at least one other species, such as 2, for example 3, such as more than 3 other species. The generic term amino acid comprises both natural and non-natural amino acids any of which may be in the "D" or "L" isomeric form.

Diterpene synthases (diTPS): Diterpene synthases (diTPS, EC 4.2.3.X) are enzymes capable of synthesising diterpene olefins and alcohols by sequential cycloisomerisation of the substrate geranylgeranyl-diphosphate (GGPP). DiTPS can be sorted in two classes, depending on the presence of a conserved motif. Class I diTPS contain an active site with a DDxxD motif, where D is an aspartic acid and x is any amino acid. Class II diTPS contain an active site with a DxDD motif, where D is an aspartic acid and x is any amino acid. Bifunctional classI/II diTPS contain two active sites, with a DDxxD and a DxDD motif, respectively.

Diterpenoid: As used herein, a diterpenoid is an unsaturated hydrocarbon based on the isoprene unit (C₅H₈), and having a general formula C_{5X}H_{8X}. A diterpene contains a backbone of 20 carbon atoms, which can be decorated by additional groups, e.g. by esterification. A diterpenoid also is a type of diterpene. A diterpenoid can derive from geranylgeranyl pyrophosphate (GGPP). Diterpenoids include all types of molecules derived from GGPP with a very broad range of functionalization. Examples of diterpenoids are olefins and diterpene alcohols.

Enantiomer: An enantiomer or enantiomorph or epimer is one of two stereoisomers that are mirror images of each other that are non-superposable. In other words, an enantiomer is a chiral molecule having a non-superposable mirror image. Enantiomers have, when present in a symmetric environment, identical chemical and physical properties except for their ability to rotate plane-polarized light (+/-) by equal amounts but in opposite directions. Enantiomers of one compound often react differently with other substances that are also enantiomers. Since many molecules in the living organisms are enantiomers themselves, there is sometimes a marked difference in the effects of two enantiomers on these organisms. In drugs, for example, often only one of a drug's enantiomers is responsible for the desired physiologic effects, while the other enantiomer is less active, inactive, or sometimes even responsible for adverse effects. Thus drugs composed of only one enantiomer (enantiomerically pure) can be developed to enhance the pharmacological efficacy and possibly dampen some side effects.

Enantiomerically pure: Enantiomerically pure, or enantiopure, refers to samples having, within the limits of detection, molecules of only one chirality.

Fragment: is used to indicate a non-full length part of a polynucleotide or polypeptide. Thus, a fragment is itself also a polynucleotide or polypeptide, respectively.

Identity: The determination of percent identity between sequences such as polynucleotide sequences or amino acid sequences can be accomplished using a mathematical algorithm. A preferred, non-limiting example of a mathematical algorithm utilized for the comparison of two sequences is the algorithm of Karlin and Altschul (1990) Proc. Natl. Acad. Sci. USA 87:2264-2268, modified as in Karlin and Altschul (1993) Proc. Natl. Acad. Sci. USA 90:5873-5877. Such an algorithm is incorporated into the BLASTN and BLASTP programs of Altschul, et al. (1990) J. Mol. Biol. 215:403-410. In order to characterize the identity, subject sequences are aligned so that the highest order homology (match) is obtained. Based on these general principles, the "percent identity" of two polynucleotide sequences may be determined using the BLASTN algorithm [Tatiana A. Tatusova, Thomas L. Madden: Blast 2 sequences - a new tool for comparing protein and nucleotide sequences; FEMS Microbiol. Lett. 1999 174 247-250], which is available from the National Center for Biotechnology Information (NCBI) web site (http://www.ncbi.nlm.nih.gov), and using the default settings suggested here (i.e. Reward for a match = 1; Penalty for a mismatch = -2; Strand option = both strands; Open gap = 5; Extension gap = 2; Penalties gap x_dropoff = 50; Expect = 10; Word size = 11; Filter on). The BLASTN algorithm determines the % sequence identity in a range of overlap between two aligned nucleotide sequences. Another preferred, non-limiting example of a mathematical algorithm utilized for the comparison of sequences is the CLUSTAL W (1.7) alignment algorithm (Thompson, J.D., Higgins, D.G. and Gibson, T.J. (1994) CLUSTAL W: improving the sensitivity of progressive multiple sequence alignment through sequence weighting, positions-specific gap penalties and weight matrix choice. Nucleic Acids Research, 22:4673-4680.). CLUSTAL W can be used for multiple sequence alignment preferably using BLOSUM 62 as scoring matrix. When calculating sequence identities, CLUSTAL W includes any gaps made by the alignment in the length of the reference sequence. Sequence identities are calculated by dividing the number of matches by the length of the aligned sequences with gaps.

Operative: An operative domain in relation to a class I or class II domain refers to a domain securing the biological processing of the polypeptide.

Plastidial targeting signal: A short sequence of amino acids that determines that a polypeptide should locate to the plastid in a plant cell.

Polynucleotide: A chain or sequence of nucleotides that convey genetic information. In regards to the present invention the polynucleotide is a deoxyribonucleic acid (DNA). Polypeptide: Plurality of covalently linked amino acid residues defining a sequence and linked by amide bonds. The term is used analogously with oligopeptide and peptide. The natural and/or non-natural amino acids may be linked by peptide bonds or by non-peptide bonds. The term peptide also embraces post-translational modifications introduced by chemical or enzyme-catalyzed reactions, as are known in the art. The term can refer to a variant or fragment of a polypeptide.

Promoter: A binding site in a DNA chain at which RNA polymerase binds to initiate transcription of messenger RNA by one or more nearby structural genes. An inducible promoter refers to a promoter where initiation of transcription can be induced by e.g. addition of a compound to the growth medium or by changing the temperature.

Racemic mixture: A racemic mixture contains equal parts of an optically active isomer and its enantiomer and has zero net rotation of plane-polarized light.

Substantially pure: As used herein, substantially pure means sufficiently homogeneous to appear free of readily detectable impurities as determined by standard methods of analysis, such as thin layer chromatography (TLC), gel electrophoresis and high performance liquid chromatography (HPLC), gas-chromatography mass-spectrometry (GC-MS), used by those of skill in the art to assess such purity, or sufficiently pure such that further purification would not detectably alter the physical and chemical properties, such as enzymatic and biological activities, of the substance. Methods for purification of the compounds to produce substantially chemically pure compounds are known to those of skill in the art. A substantially chemically pure compound can, however, be a mixture of stereoisomers or isomers. In such instances, further purification might increase the specific activity of the compound.

Transient expression: Transient expression refers to temporary expression of a polypeptide, for a limited period of time. Transient expression can be controlled by inducible and repressible promoters, agroinfiltration of plant cells with a bacterium such as *Agrobacterium tumefaciens,* and other methods known in the art.

Variant: a 'variant' of a given reference polynucleotide or polypeptide refers to a polynucleotide or polypeptide that displays a certain degree of sequence homology/identity to said reference polynucleotide or polypeptide but is not identical to said reference polynucleotide or polypeptide.

Vector: A vector is a DNA molecule or an organism comprising a DNA molecule used as a vehicle to artificially carry foreign genetic material into another cell, where the DNA molecule can be replicated and/or expressed. The vectors herein may be plasmids, viral vectors, cosmids, bacterial vectors and artificial chromosomes.

The polypeptides of the invention are particularly relevant for the enzymatic synthesis of manoyl oxide. The polypeptides of the invention are also relevant for the enzymatic synthesis of pure enantiomers of manoyl oxide.

Manoyl oxide is a compound of general formula C₂₀H₃₄O. It has been detected as an experimental artefact in a racemic mixture of the (13R) epimer (I) and (13S) epimer (II).

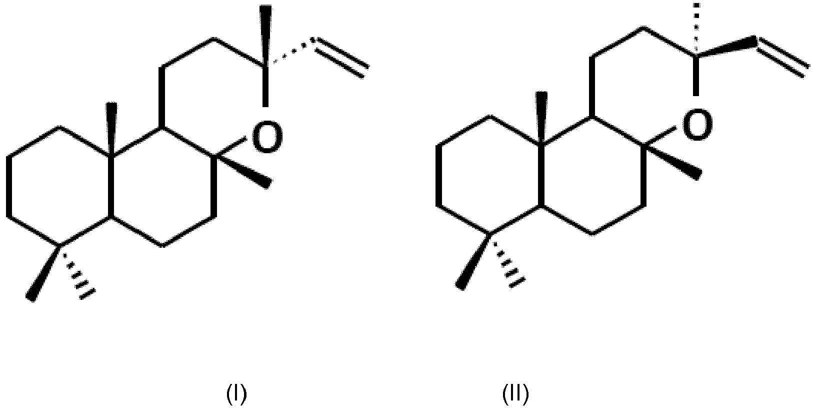

(13R) manoyl oxide has been hypothesised to be a precursor for the synthesis of forskolin.

(13R)-manoyl oxide may be synthesised stereospecifically, i.e. in an enantiomerically pure form, by the following reaction: 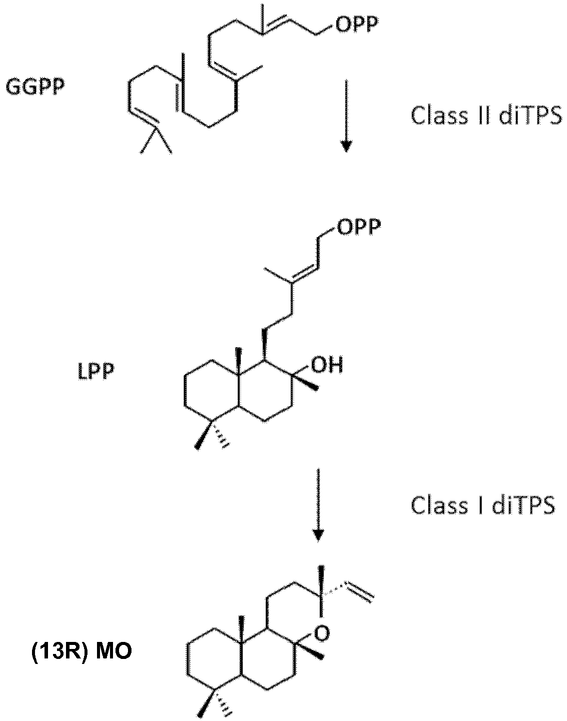 where GGPP is geranylgeranyl diphosphate, LPP is labd-13-en-8,15-diol diphosphate, and (13R) MO is (13R)-manoyl oxide.

The first step of the reaction is catalysed by a diterpene synthase (diTPS) having a class II diTPS activity. The class II diTPS catalyses protonation-initiated cationic cycloisomerization of GGPP to LPP. The reaction is terminated either by deprotonation or by water capture of the diphosphate carbocation. Class II diTPS relevant for the invention are TPS2 from *Coleus forskohlii* (CfTPS2) and LPPS from *Salvia sclarea* (SsLPPS).

The second step of the reaction is catalysed by a diTPS having a class I diTPS activity. It catalyzes cleavage of the diphosphate group of LPP and additional cyclization or rearrangement reactions on the resulting carbocation, yielding (13R)-manoyl oxide. As with the class II diTPSs, deprotonation or water capture terminate the class I diTPS reaction. Class I diTPS relevant for the invention are TSP3 and TPS4 from *Coleus forskohlii* (CfTPS3 and CfTPS4, respectively) and TPS8 from *Euphorbia peplus* (EpTPS8).

### Class I diterpene synthases

The polypeptide of the invention has a diTPS activity. In some embodiments, the polypeptide has a class I diTPS activity. Class I diTPS are capable of cleaving diphosphate groups and performing rearrangement reactions such as cyclization.

Some polypeptides of the invention have a class I diTPS activity and catalyse cleavage of the diphosphate group of LPP and additional cyclization or rearrangement reactions on the resulting carbocation, yielding (13R)-manoyl oxide. Deprotonation or water capture terminate the class I diTPS reaction. Class I diTPS relevant for the invention are TSP3 and TPS4 from *Coleus forskohlii* (CfTPS3 and CfTPS4, respectively) and TPS8 from *Euphorbia peplus* (EpTPS8).

In one embodiment the polypeptide of the invention comprises:
i) an amino acid sequence identical to SEQ ID NO: 2 [CfTPS3],
ii) a biologically active sequence variant of said polypeptide, wherein the sequence variant is at least 75% identical to, such as at least 80% identical to, such as at least 85% identical to, such as at least 90% identical to, such as at least 95% identical to, such as at least 96% identical to, such as at least 97% identical to, such as at least 98% identical to, such as at least 99% identical to, such as 100% identical to SEQ ID NO: 2 [CfTPS3],
iii) a biologically active fragment of at least 50 contiguous amino acids of any of i) through ii), said fragment having at least 75% sequence identity to SEQ ID NO: 2 [CfTPS3] in a range of overlap of at least 50 amino acids, wherein the biological activity is diterpene synthase activity, preferably class I diterpene synthase activity.

In a preferred embodiment, the polypeptide comprises a biologically active sequence variant having an amino acid sequence at least 95% identical to SEQ ID NO: 2 [CfTPS3]. In another preferred embodiment, the polypeptide comprises an amino acid sequence 100% identical to SEQ ID NO: 2 [CfTPS3]. The polypeptide of the invention may comprise a biologically active fragment of at least 50 contiguous amino acids of any of i) through ii), said fragment having at least 75% sequence identity to SEQ ID NO: 2 [CfTPS3] in a range of overlap of at least 50 amino acids, wherein the biological activity is diterpene synthase activity, preferably class I diterpene synthase activity.

In another embodiment the polypeptide of the invention comprises:
i) an amino acid sequence identical to SEQ ID NO: 3 [CfTPS4],
ii) a biologically active sequence variant of said polypeptide, wherein the sequence variant is at least 75% identical to, such as at least 80% identical to, such as at least 85% identical to, such as at least 90% identical to, such as at least 95% identical to, such as at least 96% identical to, such as at least 97% identical to, such as at least 98% identical to, such as at least 99% identical to, such as 100% identical to SEQ ID NO: 3 [CfTPS4],
iii) a biologically active fragment of at least 50 contiguous amino acids of any of i) through ii), said fragment having at least 75% sequence identity to SEQ ID NO: 3 [CfTPS4] in a range of overlap of at least 50 amino acids, wherein the biological activity is diterpene synthase activity, preferably class I diterpene synthase activity.

In a preferred embodiment, the polypeptide comprises a biologically active sequence variant having an amino acid sequence at least 95% identical to SEQ ID NO: 3 [CfTPS4]. In another preferred embodiment, the polypeptide comprises an amino acid sequence 100% identical to SEQ ID NO: 3 [CfTPS4]. The polypeptide of the invention may comprise a biologically active fragment of at least 50 contiguous amino acids of any of i) through ii), said fragment having at least 75% sequence identity to SEQ ID NO: 3 [CfTPS4] in a range of overlap of at least 50 amino acids, wherein the biological activity is diterpene synthase activity, preferably class I diterpene synthase activity.

In yet another embodiment, embodiment the polypeptide of the invention comprises:
i) an amino acid sequence identical to SEQ ID NO: 4 [EpTPS8],
ii) a biologically active sequence variant of said polypeptide, wherein the sequence variant is at least 75% identical to, such as at least 80% identical to, such as at least 85% identical to, such as at least 90% identical to, such as at least 95% identical to, such as at least 96% identical to, such as at least 97% identical to, such as at least 98% identical to, such as at least 99% identical to, such as 100% identical to SEQ ID NO: 4 [EpTPS8],
iii) a biologically active fragment of at least 50 contiguous amino acids of any of i) through ii), said fragment having at least 75% sequence identity to SEQ ID NO: 4 [EpTPS8] in a range of overlap of at least 50 amino acids, wherein the biological activity is diterpene synthase activity, preferably class I diterpene synthase activity.

In some embodiments, the polypeptide of the invention comprises an operative class I DDxxD (SEQ ID NO: 10) domain. Preferably, the polypeptides of the invention or variants or fragments thereof as defined above comprise a DDxxD domain. This domain is found at positions 531-535 of SEQ ID NO: 3 [CfTPS4], 299-303 of SEQ ID NO: 2 [CfTPS3], 540-544 of SEQ ID NO: 4 [EpTPS8]. Other important functional domains of the class I diTPS polypeptides of the invention are Mg²⁺-binding sites, active site lid residues and substrate binding pockets. The relevant residues for each of SEQ ID NO: 2 [CfTPS3], SEQ ID NO: 3 [CfTPS4] and SEQ ID NO: 4 [EpTPS8] are listed in Table 1. Some embodiments of the invention concern polypeptides in which these residues or domains are not modified.

In some embodiments, the polypeptides of the invention comprise a plastidial target domain. The plastidial target domain is comprised in the domain ranging from positions 1 to 73 of SEQ ID NO: 3 [CfTPS4], 1 to 73 of SEQ ID NO: 2 [CfTPS3], 1 to 73 of SEQ ID NO: 4 [EpTPS8], respectively. Thus preferred polypeptides comprise the plastidial target domain.

Table 1. Important domains and residues for class I diTPS of the invention. The residues are indicated as Xn, where X is the one letter amino acid code and n the position of the residue in the relevant sequence, except for the plastidial targeting signal, where the two numbers indicate the positions of the first and last residue of the predicted domain.

| | Plastidial targeting signal | Mg²⁺-binding sites | Active site lid residues | Substrate binding pockets |
|---|---|---|---|---|
| CfTPS3 (SEQ ID NO: 2) | 1-73 | N443, E451 | EKERKENTGNSV (449-460); GDEF (526-529) | R262 L271 H292 V294 L295 T297 D300 D304 R440 L441 N443 D444 T446 E451 Y523 G526 F529 |
| CfTPS4 (SEQ ID NO: 3) | 1-73 | N475, E483 | EKERKENTGNSV (481-492); GDEF (559-562) | R294 L303 H324 V326 L327 T329 D332 D336 R472 L473 N475 D476 T478 E483 Y556 G559 F562 |
| EpTPS8 (SEQ ID NO: 4) | 1-73 | N684, E692 | KRESAQGKLNGV (690-701); DDGF (536-538) | R503 T512 N533 V535 F536 T538 D541 D545 R681 L682 N684 D685 T687 E692 Y764 D767 F770 |

Mg²⁺-binding sites for CfTPS3 (SEQ ID NO: 2) are found at positions 443 (N) and 451 (E).

### Class II diterpene synthases

The polypeptide of the invention has a diTPS activity. In some embodiments, the polypeptide has a class II diTPS activity. Class II diTPS are capable of catalysing protonation-initiated cationic cycloisomerization reactions. The reaction is terminated either by deprotonation or by water capture of the diphosphate carbocation. A class II diTPS relevant for the invention is TPS2 from *Coleus forskohlii* (CfTPS2), which can catalyse cycloisomerisation of GGPP to LPP. Deprotonation or water capture terminate the class II diTPS reaction.

In one embodiment the polypeptide of the invention comprises:
i) an amino acid sequence identical to SEQ ID NO: 1 [CfTPS2],
ii) a biologically active sequence variant of said polypeptide, wherein the sequence variant is at least 75% identical to, such as at least 80% identical to, such as at least 85% identical to, such as at least 90% identical to, such as at least 95% identical to, such as at least 96% identical to, such as at least 97% identical to, such as at least 98% identical to, such as at least 99% identical to, such as 100% identical to SEQ ID NO: 1 [CfTPS2],
iii) a biologically active fragment of at least 50 contiguous amino acids of any of i) through ii), said fragment having at least 75% sequence identity to SEQ ID NO: 1 [CfTPS2] in a range of overlap of at least 50 amino acids, wherein the biological activity is diterpene synthase activity, preferably class I diterpene synthase activity.

In a preferred embodiment, the polypeptide comprises a biologically active sequence variant having an amino acid sequence at least 95% identical to SEQ ID NO: 1 [CfTPS2]. In another preferred embodiment, the polypeptide comprises an amino acid sequence 100% identical to SEQ ID NO: 1 [CfTPS2]. The polypeptide of the invention may comprise a biologically active fragment of at least 50 contiguous amino acids of any of i) through ii), said fragment having at least 75% sequence identity to SEQ ID NO: 1 [CfTPS2] in a range of overlap of at least 50 amino acids, wherein the biological activity is diterpene synthase activity, preferably class I diterpene synthase activity.

In some embodiments, the polypeptide of the invention comprises an operative class II DxDD domain. Preferably, the polypeptides of the invention or variants or fragments thereof as defined above comprise a DxDD domain. This domain is found at positions 358-361 of SEQ ID NO: 1 [CfTPS2].

In some embodiments, the polypeptides of the invention comprise a plastidial target domain. The plastidial target domain is comprised in the domain ranging from positions 1 to 73 of SEQ ID NO: 1 [CfTPS2].

### Polynucleotide

The invention further relates to a polynucleotide encoding a polypeptide according to the invention.

In some embodiments, the polynucleotide has a sequence with at least 85% identity to a sequence selected from the group consisting of SEQ ID NO: 5 [CfTPS2], SEQ ID NO: 6 [CfTPS3], SEQ ID NO: 7 [CfTPS4] and SEQ ID NO: 8 [EpTPS8]. In some embodiments, the polynucleotide has a sequence with at least 85% identity, such as at least 90% identity, such as at least 95% identity, such as at least 96% identity, such as at least 97% identity, such as at least 98% identity, such as 99% identity, such as 100% identity to SEQ ID NO: 5 [CfTPS2]. In other embodiments, the polynucleotide has a sequence with at least 85% identity, such as at least 90% identity, such as at least 95% identity, such as at least 96% identity, such as at least 97% identity, such as at least 98% identity, such as 99% identity, such as 100% identity to SEQ ID NO: 6 [CfTPS3]. In other embodiments, the polynucleotide has a sequence with at least 85% identity, such as at least 90% identity, such as at least 95% identity, such as at least 96% identity, such as at least 97% identity, such as at least 98% identity, such as 99% identity, such as 100% identity to SEQ ID NO: 7 [CfTPS4]. In other embodiments, the polynucleotide has a sequence with at least 85% identity, such as at least 90% identity, such as at least 95% identity, such as at least 96% identity, such as at least 97% identity, such as at least 98% identity, such as 99% identity, such as 100% identity to SEQ ID NO: 8 [EpTPS8]. Thus the polynucleotide s of the invention encode polypeptides having a diTPS activity as described above.

Preferably, the polynucleotide comprises a sequence coding for an operative class I domain DDxxD. The DDxxD domain of the CfTPS3 diTPS is DDFFD, where D is an aspartic acid and F is a phenylalanine, and is found at positions 330 to 334 (SEQ ID NO: 2). The DDxxD domain of the CfTPS4 diTPS is DDFFD, where D is an aspartic acid and F is a phenylalanine, and is found at positions 331 to 335 (SEQ ID NO: 3). The DDxD domain of the EpTPS8 diTPS is DDFFD, where D is an aspartic acid and F is a phenylalanine, and is found at positions 540 to 544 (SEQ ID NO: 4).

In another preferred embodiment, the polynucleotide comprises a sequence coding for an operative class II domain DxDD. The DxDD domain of the CfTPS2 diTPS is DIDD, where D is an aspartic acid and I is an isoleucine residue, and is found at positions 399 to 402 (SEQ ID NO: 1).

Some polynucleotides of the invention may comprise a sequence coding for a plastidial targeting signal. The plastidial target domain is comprised in the corresponding polypeptides in the domains ranging from positions 1 to 50 of SEQ ID NO: 1 [CfTPS2], 1 to 33 of SEQ ID NO: 3 [CfTPS4], 1 to 3 of SEQ ID NO: 2 [CfTPS3], 1 to 5 of SEQ ID NO: 4 [EpTPS8], respectively.

The polynucleotide may have a sequence that is codon-optimised. Codon optimisation methods are known in the art and allow optimised expression in a heterologous host organism or cell. The host cell may be selected from the group comprising bacterial cell, yeast cells, fungal cells, plant cells, mammalian cells and insect cells. The host cell may be selected from the group comprising *Escherichia coli, Saccharomyces cerevisiae, Schizosaccharomyces pombe, Nicotiana benthamiana* and *Physcomitrella patens.* Thus in one embodiment, the host cell is *Escherichia coli.* In another embodiment, the host cell is *Saccharomyces cerevisiae* or *Schizosaccharomyces pombe.* In another embodiment, the host cell is *Nicotiana benthamiana.* In another embodiment, the host cell is *Physcomitrella patens.*

### Vectors

The invention further relates to a vector comprising at least one polynucleotide as defined above. Thus the invention relates to a vector suitable for expression of at least one polypeptide having a diTPS activity.

In some embodiments, the vector comprises a polynucleotide having a sequence with at least 85% identity to a sequence selected from the group consisting of SEQ ID NO: 5 [CfTPS2], SEQ ID NO: 6 [CfTPS3], SEQ ID NO: 7 [CfTPS4] and SEQ ID NO: 8 [EpTPS8]. In some embodiments, the vector comprises a polynucleotide having a sequence with at least 85% identity, such as at least 90% identity, such as at least 95% identity, such as at least 96% identity, such as at least 97% identity, such as at least 98% identity, such as 99% identity, such as 100% identity to SEQ ID NO: 5 [CfTPS2]. In other embodiments, the vector comprises a polynucleotide having a sequence with at least 85% identity, such as at least 90% identity, such as at least 95% identity, such as at least 96% identity, such as at least 97% identity, such as at least 98% identity, such as 99% identity, such as 100% identity to SEQ ID NO: 6 [CfTPS3]. In other embodiments, the vector comprises a polynucleotide having a sequence with at least 85% identity, such as at least 90% identity, such as at least 95% identity, such as at least 96% identity, such as at least 97% identity, such as at least 98% identity, such as 99% identity, such as 100% identity to SEQ ID NO: 7 [CfTPS4]. In other embodiments, the polynucleotide has a sequence with at least 85% identity, such as at least 90% identity, such as at least 95% identity, such as at least 96% identity, such as at least 97% identity, such as at least 98% identity, such as 99% identity, such as 100% identity to SEQ ID NO: 8 [EpTPS8].

In some embodiments, the vector comprises a first polynucleotide having a sequence with at least 85% identity to a sequence selected from the group consisting of SEQ ID NO: 5 [CfTPS2], SEQ ID NO: 6 [CfTPS3], SEQ ID NO: 7 [CfTPS4] and SEQ ID NO: 8 [EpTPS8] and a second polynucleotide having a sequence with at least 85% identity to a sequence selected from the group consisting of SEQ ID NO: 5 [CfTPS2], SEQ ID NO: 6 [CfTPS3], SEQ ID NO: 7 [CfTPS4] and SEQ ID NO: 8 [EpTPS8], where the second polynucleotide is different from the first polynucleotide. In preferred embodiments, the vector comprises a first polynucleotide coding for a polypeptide with a class I diTPS activity and a second polynucleotide coding for a polypeptide with a class II diTPS activity. In a preferred embodiment, the first polynucleotide has a sequence with at least 85% identity to SEQ ID NO: 5 [CfTPS2], and the second polynucleotide has a sequence with at least 85% identity to SEQ ID NO: 6 [CfTPS3], SEQ ID NO: 7 [CfTPS4] and SEQ ID NO: 8 [EpTPS8].

Vectors of the invention comprise plasmids, cosmids, viral vectors, artificial chromosomes and bacterial vectors. The vector may be suitable for transient expression of the at least one polynucleotide. Such vectors are known in the art. For example, expression may be induced by addition of a compound to the growth medium. The vector may be a bacterial vector, such as *Agrobacterium tumefaciens.* In a preferred embodiment, the vector also encodes a viral suppressor of gene silencing, such as the p19 protein of tomato bushy stunt virus.

### Host cell

The invention further relates to a host cell comprising a polynucleotide as defined above and/or a vector as defined above. In some embodiments, the host cell is capable of producing (13R)-manoyl oxide.

The host cell of the invention is capable of expressing:
(i) a first polypeptide having a sequence at least 70% identical to SEQ ID NO: 1 [CfTPS2]; and
(ii) a second polypeptide having a sequence at least 70% identical to SEQ ID NO: 2 [CfTPS3], SEQ ID NO: 3 [CfTPS4], or SEQ ID NO: 4 [EpTPS8].

In some embodiments, the host cell is capable of expressing a first polypeptide having a class I diTPS activity and a second polypeptide having a class II diTPS activity.

Thus in some embodiments the host cell is capable of expressing a first polypeptide having a sequence at least 70% identical to SEQ ID NO: 1 [CfTPS2] and a second polypeptide having a sequence at least 70% identical to SEQ ID NO: 2 [CfTPS3].

In other embodiments, the host cell is capable of expressing a first polypeptide having a sequence at least 70% identical to SEQ ID NO: 1 [CfTPS2] and a second polypeptide having a sequence at least 70% identical to SEQ ID NO: 3 [CfTPS4].

In other embodiments, the host cell is capable of expressing a first polypeptide having a sequence at least 70% identical to SEQ ID NO: 1 [CfTPS2] and a second polypeptide having a sequence at least 70% identical to SEQ ID NO: 4 [EpTPS8].

The host cell may be selected from the group comprising bacterial cell, yeast cells, fungal cells, plant cells, mammalian cells and insect cells. The host cell may be selected from the group comprising *Escherichia coli, Saccharomyces cerevisiae, Schizosaccharomyces pombe, Nicotiana benthamiana* and *Physcomitrella patens.* Thus in one embodiment, the host cell is *Escherichia coli.* In another embodiment, the host cell is *Saccharomyces cerevisiae* or *Schizosaccharomyces pombe.* In another embodiment, the host cell is *Nicotiana benthamiana.* In another embodiment, the host cell is *Physcomitrella patens.*

Methods for expressing the first and second polypeptides in the host cell are known in the art. One or both of the first and second polypeptides may be heterologously expressed from polynucleotide sequences cloned into the genome of the host cell or they may be comprised within a vector as described above. For example, a first polynucleotide coding for the first polypeptide is cloned into the genome, and a second polynucleotide coding for the second polypeptide is comprised within a vector transformed or transfected into the host cell. Alternatively, the first polynucleotide is comprised within a first vector and the second polynucleotide is comprised within a second vector. The first and second vector may be one vector. Vectors suitable for expression of the first and second polypeptides are known in the art.

Expression of the first and second polypeptides in the host cell may occur in a transient manner. When the polynucleotide encoding one of the polypeptides is cloned into the genome, an inducible promoter may be cloned as well to control expression of the polypeptides. Such inducible promoters are known in the art. Alternatively, genes coding for suppressors of gene silencing may also be cloned in the genome or into a vector transfected within the host cell.

The host cell may be selected from the group comprising bacterial cell, yeast cells, fungal cells, plant cells, mammalian cells and insect cells. The host cell may be selected from the group comprising *Escherichia coli, Saccharomyces cerevisiae, Schizosaccharomyces pombe, Nicotiana benthamiana* and *Physcomitrella patens.* Thus in one embodiment, the host cell is *Escherichia coli.* In another embodiment, the host cell is *Saccharomyces cerevisiae* or *Schizosaccharomyces pombe.* In another embodiment, the host cell is *Nicotiana benthamiana.* In another embodiment, the host cell is *Physcomitrella patens.*

In some embodiments, the host cell is transfected with a vector. The vector may be selected from the group comprising plasmids, cosmids, viral vectors, artificial chromosomes and bacterial vectors. The vector may be constructed so that transient expression of the first and/or second polynucleotide from the vector is transient. Transient expression from the vector may occur via induction of an inducible promoter as is known in the art. In a preferred embodiment, the host cell is a *Nicotiana benthamiana* cell, such as a leaf cell. In another preferred embodiment, the vector is the bacterial vector *Agrobacterium tumefaciens.* In a preferred embodiment, the vector also encodes a viral suppressor of gene silencing. In a most preferred embodiment, the vector encodes the p19 protein from tomato bushy stunt virus.

The host cell may naturally be capable of producing GGPP. In other embodiments, the cell may be engineered so that it is capable of producing GGPP. For example, the plastidial methylerythritol 4-phosphate (MEP) pathway may be cloned into the host cell. The host cell may be further engineered in order to redirect metabolic fluxes and optimise for the production of a specific compound.

In some embodiments, the host cell is capable of producing diterpenoids. The host cell may be capable of producing (13R)-manoyl oxide in a substantially pure enantiomeric form.

### Examples

### Example 1: Identification of Coleus forskohlii diTPS genes.

Forskolin is a representative of an unusual series of tricyclic (8,13)-epoxy-labdanes, characteristic for this plant. Given its importance as a pharmaceutical, we set out to discover genes involved in the biosynthesis of forskolin.

### Materials and methods

### Plant Growth and Microscopy

*Coleus forskohlii* (Lamiaceae) plants were grown in the greenhouse at the University of Copenhagen, Denmark, under ambient photoperiod and 24°C day/17°C night temperatures. Transverse sections of roots (diameter of approximately 1 to 5 mm) were prepared for histochemical analysis. Sections were observed unstained with a Leica DM 5000B or a Nikon Eclipse 80i light and fluorescence microscope.

Additionally, root samples were fixed in a solution containing 2.5% glutaraldehyde, 2% paraformaldehyde, and 0.1 M sodium cacodylate buffer, pH 7.2 for 24 h, thereafter surface sections and cross sections from the root cork were incubated in 0.1 µg/mL Nile Red for identification of lipid components. Images from intact cells were recorded in a Leica SP5X confocal laser scanning microscope (Leica Microsystems, Mannheim, Germany).

### Diterpene profiling and forskolin quantification in C. forskohlii tissues

Tissue was extracted. Cold methanol acidified with formic acid (0.125%) was added to ground and frozen tissue samples in a ratio of 3:1 (solvent:tissue). Samples were sonicated in an ultrasonic bath at 23°C for 15 min at 40 kHz (Branson, 3510), filtered using 96 well filter plates and analyzed by HPLC (High-Performance Liquid Chromatography) equipped with an evaporative light scattering detector (ELSD). All tissue types were extracted in triplicate. Forskolin was quantified by comparison to a standard series of forskolin (Sigma) which was prepared as per the tissue samples.

For the diterpene profiling of the root cork HPLC-MS, extracts were prepared as for forskolin quantification. Separation was carried out on an Agilent 1100 Series LC unit (Agilent Technology) with column and gradient as described above. The LC unit was coupled to either a Bruker microTOF mass spectrometer (Bruker Daltronics) for high resolution mass measurements or a Bruker HCT-Ultra ion trap mass spectrometer (Bruker Daltronics) for MSn experiments.

### Isolation of C. forskohlii root cork oil bodies

For isolation of oil bodies from root cork tissue, approximately 15 g of tissue was gently ground in 100 mL extraction buffer (EB; 20 mM Tricine, 250 mM sucrose, 0.2 mM PMSF, pH 8.5); the homogenate was filtered through Miracloth (Calbiochem) and centrifuged at 3500 rpm for 10 min for separation of cellular debris. The supernatant was collected and transferred in centrifugation tubes. Buffer B (20% sucrose, 20 mM HEPES, 100 mM KCI, 2 mM MgCl₂, pH 10.5) was overlaid (5 mL of B for 25 mL of supernatant) and samples were centrifuged for 40 min at 5000 g. The resulting floating oil bodies were collected carefully from the surface layer.

### Identification and cloning of full length diTPS genes

Mining of the *C*. *forskohlii* databases was performed as previously described (Zerbe et al., 2013), using tBLASTn software and known angiosperm diTPSs as query (CPS and EKS) and guided full-length cloning of a number of putative class I and class II diTPS genes. Total RNA from *C*. *forskohlii* roots, extracted as previously described (Hamberger et al., 2011), was used for cDNA synthesis. Cloning of the putative diTPS genes was achieved after PCR amplification using gene specific primers amplification using gene specific primers (SEQ ID NO: 11 to 40) that were designed based on the *in silico* sequences of the identified CfTPS genes.

### RNA extraction and Quantitative Real-Time PCR

Total RNA from C. *forskohlii* root cork was extracted according to Hamberger et al., 2011, and further purified using the Spectrum Plant Total RNA Kit (Sigma) while total RNA from leaves, flowers, stems and root cortex and stele was extracted using the Spectrum Plant Total RNA Kit (Sigma). RNA extraction was followed by on-column DNase I digestion. First-strand cDNAs were synthesized from 0.5 µg of total RNA. The resulting cDNA was diluted 10-fold for the qRT-PCR reactions. Quantitative real-time PCR reactions were performed with gene specific primers (SEQ ID NO: 41 to 60) and Maxima SYBR Green/Fluorescein qPCR Master Mix (Fermentas) on a Rotor-Gene Q cycler (Qiagen). TIF4a and EF1a were used as reference genes as they showed the lowest variation across different tissues. The results were normalized with TIF4a. Relative transcript abundance was calculated as the mean of three biological replications (three different plants), while the reactions were performed in three technical replicates. Amplification efficiency was calculated with the "Real Time PCR Miner" (http://www.miner.ewindup.info/Version2). Efficiency-corrected ΔC_{T} values were used to quantify relative differences in target gene transcript accumulation.

### Functional characterization of CfTPS - in vitro assays

For the expression of CfTPS1, CfTPS2, CfTPS3, CfTPS4 and CfTPS14 in *E. coli,* pseudomature variants lacking predicted plastidial target sequences were cloned into the into pET28b+ vector. The software ChloroP was used for prediction of the plastidial target sequence (http://www.cbs.dtu.dk/services/ChloroP/). As the expression levels of the recombinant CfTPS3 were very poor, a codon optimized version was synthesized by GenScript USA Inc. and subsequently cloned into the same vector (SEQ ID NO: 61). pET28b+ constructs were transformed into *E. coli* BL-21 DE3-C41 cells and and grown in selection medium until the OD₆₀₀ reached 0.3-0.4. Expression was induced at OD₆₀₀ ∼0.6 with 0.2 mM IPTG. Expression was done overnight and cells were harvested by centrifugation and lysed. The cell lysates were centrifuged and the supernatant was subsequently used for purification of the recombinant proteins. CfTPS proteins were purified on 1 mL His SpinTrap™ columns (GE healthcare). *In vitro* CfTPS assays were performed by adding 15 µM GGPP and 100 µg purified CfTPS enzymes in 397 µL enzyme assay buffer (50 mM HEPES (pH 7.2), 7.5 mM MgCl₂, 5% (v/v) glycerol, 5 mM DTT). Onto the reaction mix, 500 µL n-hexane (Fluka GC-MS grade) was gently added as an overlay. Assays were incubated for 60 min at 30°C and -70 rpm and the hexane overlay was subsequently removed for GC-MS analysis.

### Functional characterization of CfTPS - transient expression in Nicotiana benthamiana

Full length CfTPS cDNAs were cloned into the agrobacterium binary vector for plant transformation pCAMBIA1300_35Su. Transient expression of *CfTPSs* with the gene silencing suppressor p19 protein in *N. benthamiana* leaves and extraction of diterpenes were performed as recently described. Hexane extracts of *N. benthamiana* expressing the gene silencing suppressor p19 protein alone were used as controls. Compounds of interest were identified by comparison of GC-MS total ion chromatogram (TIC) and extracted ion chromatograms (EIC) of 275 and 272 m/z from samples. The ion 275 m/z is characteristic of several labdane type diterpenes including manoyl oxide whereas 272 m/z is characteristic of several other non-labdane type diterpenes such as abietane like diterpenes. All extractions from *N. benthamiana* transiently expressing diTPSs were carried out in biological triplicates (different leaves/plants infiltrated with the same agrobacteria mixture).

### Metabolite analysis from in vitro and in planta assays

For the gas chromatography-mass spectrometry (GC-MS) analysis of *N. benthamiana* leaves expressing the CfTPSs and specific C. *forskohlii* tissues, 500 µL GC-MS grade hexane was added to 2 leaf discs (0=3 cm) in a 1.5 mL glass vial. After extraction, the solvent was transferred into new 1.5 mL glass vials and stored at -20°C until GC-MS analysis. Compound identification was done by comparison to authentic standards (dehydroabietadiene, abietadiene), reference spectra from literature, databases and comparison of retention time (miltiradiene, manoyl oxide, copalol, labd-13-en-8,15-diol and 13(16)-14-labdien-8-ol) (Wiley Registry of Mass Spectral Data, 8th Edition, July 2006, John Wiley & Sons, ISBN: 978-0-470-04785-9). The differentiation of the C-13 epimers (13R) and (13S)-manoyl oxide was performed as previously described (Demetzos et al., 2002).

### Accession numbers

Nucleotide sequences of characterized enzymes have been submitted to the GenBankTM/EBI Data Bank with accession numbers: CfTPS1, KF444506; CfTPS2, KF444507; CfTPS3, KF444508; CfTPS4, KF444509; CfTPS15, KF471011.

### Example 2: Localization of forskolin and (13R)-manoyl oxide in root cork oil bodies

When transverse sections of *C*. *forskohlii* root (Fig. 1A) were examined using light microscopy, we found that cells of the root cork contained oil body-like structures (hereafter termed oil bodies) with a typical distribution of one oil body per cell of the root cork (Fig. 1B). Cells containing more than one oil body were occasionally seen in older tissue (Fig. 1 C). Confocal laser scanning microscopy of *C*. *forskohlii* root cork stained with Nile Red indicated that the observed structures were indeed oil bodies and that the composition of the lipophilic content was heterogeneous, with both polar (Fig. 1 E) and neutral (Fig. 1D) lipophilic compounds, which were non-uniformly distributed. Globules of neutral lipids dispersed in predominantly polar lipids were detected by the fluorescence (Fig. 1D-F).

Separate methanol extracts of the root cork and the root stele and cortex were analysed by high-performance liquid chromatography (HPLC) using an evaporative light scattering detector (ELSD) and compared with flowers, leaves and stems. Forskolin was primarily detected in the root cork, and was not found in the root stele and cortex nor in the aerial parts of the plant (Fig. 2). To further examine if forskolin was present specifically in the oil bodies, methanol extracts of isolated oil bodies (oil bodies purified to apparent homogeneity) were subjected to HPLC-ELSD analysis, targeting polar constituents, while non-polar hexane extracts were analyzed by gas chromatography-mass spectrometry (GC-MS) (Fig. 3). In addition to forskolin, which was present in the polar fraction, we detected (13R)-manoyl oxide in the non-polar fraction.

These results demonstrate that (13R)-manoyl oxide and forskolin are present in the oil bodies of *C*. *forskohlii.*

### Example 3: C. forskohlii diTPSs constitute a small gene family specific for Lamiaceae

We mined the root transcriptome of *C*. *forskohlii* for the identification of *CfdiTPS* candidates as described (Zerbe et al., 2013). A panel of six diterpene synthases was identified, *CfTPS1, CfTPS2, CfTPS3, CfTPS4, CfTPS14* and *CfTPS15* which, with exception of *CfTPS15,* represented full-length cDNAs with predicted N-terminal plastidial transit peptides. *CfTPS1, CfTPS2,* and *CfTPS15* contained the Asp-rich conserved motif DxDD characteristic of class II diTPS, while *CfTPS3, CfTPS4* and *CfTPS14* carried the DDxxD signature motif of class I diTPS. We performed separate phylogenetic analyses of class II and class I CfTPSs including functionally characterized representatives from the Lamiaceae and other angiosperm species. Included in the phylogenies were representative gymnosperm class II and class I diTPSs [PgCPS and PgEKS] and the bifunctional diTPS from the moss *Physcomitrella patens* [PpCPS/EKS], as it is considered an ancestral archetype of plant diTPSs (Fig. 4).

Thus we demonstrate that *CfTPS1, CfTPS2,* and *CfTPS15* are phylogenetically related to class II diTPS and that *CfTPS3*, *CfTPS4* and *CfTPS14* are phylogenetically related to class I diTPS.

### Example 4: Transcript levels of C. forskohlii diTPSs in various tissues and in vitro functional characterization

To correlate the transcript levels of *CfTPS* genes with accumulation of forskolin related labdane-diterpenoids and abietane diterpenoids in C. *forskohlii* tissues, quantitative (q)RT-PCR analysis was performed using cDNA templates derived from the root cork (Ck), root cortex and stele (root without cork) (CS), leaves (Lv), stems (St) and flowers (FI) total RNA. *CfTPS1, CfTPS2* and *CfTPS3* shared a similar pattern of transcript profiles across all tissues, showing high transcript accumulation in root cork cells, up to 1000-fold in comparison with all other tissues tested (Fig. 5). These data supported involvement of *CfTPS1*, *CfTPS2* and *CfTPS3* in the formation of specialized metabolites in the root cork. In contrast, the transcript levels of *CfTPS4, CfTPS14* and *CfTPS15* were relatively low across all tissues tested. Despite the close phylogenetic relation of *CfTPS3* and *CfTPS4* (Fig. 4), they show surprisingly different expression patterns. In contrast to *CfTPS3*, *CfTPS4* transcripts were mostly detected in the aerial parts of the plant, especially in the leaves, while its transcripts accumulate only to very low levels in the root.

For the functional characterization of the CfTPSs described here (except for CfTPS15, for which no full length sequence could be retrieved), cDNAs were heterologously expressed in *E. coli* with a C-terminal 6xHis epitope tag. Purified recombinant proteins were tested individually in single or coupled *in vitro* assays, supplied with appropriate substrates and the reaction products were analyzed by GC-MS. Products of the *in vitro* assays with the class II diTSPs, CfTPS1 and CfTPS2, were treated with alkaline phosphatase before GC-MS analysis.

Enzyme assays with CfTPS1 yielded a diterpene with a mass spectrum matching copal-15-ol (h), indicating that the primary product before dephosphorylation is copalyl diphosphate (Fig. 6A). Assays of CfTPS2 resulted in the formation of 13(16)-14-labdien-8-ol (f) and labd-13-en-8,15-diol (g) as major products (Fig. 6B), supporting a function as labda-13-en-8-ol (or copal-8-ol) diphosphate synthase, similar to the functions of previous reported similar enzymes. We also detected the non-stereoselective formation of the (13R) and (13S) epimers of manoyl oxide, which were previously observed in *in vitro* reactions of similar class II diTPSs and were suggested to be the result of a non-enzymatic reaction (Caniard et al., 2012; Zerbe et al., 2013). These results indicate that CfTPS1 and CfTPS2 represent functionally distinct class II diTPSs, both necessary and sufficient to form the diphosphate intermediates required for the abietane and labdane classes of diterpenoids detected in *C*. *forskohlii.*

Assays of CfTPS1 coupled to either CfTPS3 or CfTPS4 resulted in formation of miltiradiene (d) (Fig. 6A), a labdane diterpene formed from a copalyl diphosphate intermediate (Gao et al., 2009) and is consistent with the results of the single enzyme assay of CfTPS1. Coupled assays with CfTPS2 and CfTPS3 showed the formation of the pure (13R) enantiomer of manoyl oxide (a) (Fig. 6B). In coupled assays of CfTPS2 with CfTPS4, both (13R) and (13S)-manoyl oxide epimers were detected, albeit at a ratio different from the dephosphorylation product of CfTPS2 alone (Fig. 6B). The (13R)-manoyl oxide epimer was produced stereospecifically in the combination of class II CfTPS2 and class I CfTPS3 enzymes.

These results demonstrate that the CfTPSs can be expressed heterologously in *Escherichia coli.* We further show that (13R)-manoyl oxide can be produced stereospecifically in a substantially pure enantiomeric form in an *in vitro* system.

### Example 5: In planta heterologous expression and functional characterization of C. forskohlii diTPSs

The CfTPSs were expressed in *Nicotiana benthamiana* leaves after agroinfiltration. GC-MS analyses of extracts from *N. benthamiana* leaves transiently expressing the individual class I CfTPS3, CfTPS4 and CfTPS14 did not result in detectable accumulation of additional metabolites compared to control plants (data not shown). Extracts from *N. benthamiana* expressing the class II CfTPS1 alone showed only trace amounts of additional diterpenes compared to the controls, none of which could be accurately identified (Fig. 7B).

Consistent with the *in vitro* enzyme assays, both (13R) and (13S) epimers of manoyl oxide were identified in the extracts from *N. benthamiana* expressing the class II CfTPS2 (Fig. 7A). Co-expression of CfTPS2 and CfTPS14 did not change the product profile compared to expression of CfTPS2 alone, suggesting that CfTPS14 does not accept the copal-8-ol diphosphate as substrate (Fig. 7A).

In extracts of plants co-expressing CfTPS1 with CfTPS3 or CfTPS4 miltiradiene (d) was observed as the main product together with minor traces of dehydroabietadiene (c) and abietadiene (e) (Fig. 7B). All three diterpenes were subsequently identified in stem and root tissues of *C*. *forskohlii.* While dehydroabietadiene was found in both tissues, abietadiene was mainly detected in the root cork tissue, and miltiradiene in the stem and leaf tissue of *C*. *forskohlii.*

In extracts from *N. benthamiana* co-expressing CfTPS2 with CfTPS3 or CfTPS4, only the (13R) epimer of manoyl oxide (a) was identified (Fig. 7A), consistent with the stereochemical conformation of forskolin and the related series of labdane-type diterpenoids. This result suggests that the class I CfTPS3 and CfTPS4 can accept the copal-8-ol diphosphate synthesized by CfTPS2 and catalyze the stereospecific formation of (13R)-manoyl oxide.

These results demonstrate that the CfTPSs can be agroinfiltrated in a plant organism and that (13R)-manoyl oxide can be produced stereospecifically in a substantially pure enantiomeric form in an *in vivo* system.

### References

Caniard et al., 2012. Discovery and functional characterization of two diterpene synthases for sclareol biosynthesis in Salvia sclarea (L.) and their relevance for perfume manufacture. BMC Plant Biol. 12:119.
Demetzos et al., 2002. A simple and rapid method for the differentiation of C-13 manoyl oxide epimers in biologically important samples using GC-MS analysis supported with NMR spectroscopy and computational chemistry results. Bioorg Med Chem Lett. 12(24):3605-9.
Günnewich et al., 2013. A diterpene synthase from the clary sage Salvia sclarea catalyzes the cyclization of geranylgeranyl diphosphate to (8R)-hydroxy-copalyl diphosphate. Phytochemistry 91:93-9
Hamberger et al., 2011. Evolution of diterpene metabolism: Sitka spruce CYP720B4 catalyzes multiple oxidations in resin acid biosynthesis of conifer defense against insects. Plant Physiol. 157(4):1677-95.
Zerbe et al., 2013. Gene discovery of modular diterpene metabolism in nonmodel systems. Plant Physiol. 162(2):1073-91.

### Items

1. An isolated diterpene synthase (diTPS) polypeptide comprising:
   i) an amino acid sequence selected from the group consisting of SEQ ID NO: 1 [CfTPS2], SEQ ID NO: 2 [CfTPS3], SEQ ID NO: 3 [CfTPS4] and SEQ ID NO: 4 [EpTPS8];
   ii) a biologically active sequence variant of said polypeptide, wherein the sequence variant has at least 75% sequence identity to said SEQ ID NO: 1 [CfTPS2], SEQ ID NO: 2 [CfTPS3], SEQ ID NO: 3 [CfTPS4] or SEQ ID NO: 4 [EpTPS8]; or
   iii) a biologically active fragment of at least 50 contiguous amino acids of any of i) through ii), said fragment having at least 75% sequence identity to SEQ ID NO: 1 [CfTPS2], SEQ ID NO: 2 [CfTPS3], SEQ ID NO: 3 [CfTPS4] or SEQ ID NO: 4 [EpTPS8] in a range of overlap of at least 50 amino acids,
      wherein the biological activity is diterpene synthase activity.
2. The polypeptide according to item 1, wherein the polypeptide has a class I diTPS activity.
3. The polypeptide according to any one of the preceding items, wherein the sequence is at least 75% identical to, such as at least 80% identical to, such as at least 85% identical to, such as at least 90% identical to, such as at least 95% identical to, such as at least 96% identical to, such as at least 97% identical to, such as at least 98% identical to, such as at least 99% identical to, such as 100% identical to SEQ ID NO: 2 [CfTPS3].
4. The polypeptide according to any one of the preceding items, wherein the sequence is at least 75% identical to, such as at least 80% identical to, such as at least 85% identical to, such as at least 90% identical to, such as at least 95% identical to, such as at least 96% identical to, such as at least 97% identical to, such as at least 98% identical to, such as at least 99% identical to, such as 100% identical to SEQ ID NO: 3 [CfTPS4].
5. The polypeptide according to any one of the preceding items, wherein the sequence is at least 75% identical to, such as at least 80% identical to, such as at least 85% identical to, such as at least 90% identical to, such as at least 95% identical to, such as at least 96% identical to, such as at least 97% identical to, such as at least 98% identical to, such as at least 99% identical to, such as 100% identical to SEQ ID NO: 4 [EpTPS8].
6. The polypeptide according to any one of the preceding items, comprising an operative class I DDxxD domain.
7. The polypeptide according to item 1, wherein the polypeptide has a class II diTPS activity.
8. The polypeptide according to item 7, having a sequence at least 75% identical to, such as at least 80% identical to, such as at least 85% identical to, such as at least 90% identical to, such as at least 95% identical to, such as at least 96% identical to, such as at least 97% identical to, such as at least 98% identical to, such as at least 99% identical to, such as 100% identical to SEQ ID NO: 1 [CfTPS2].
9. The polypeptide according to any one of items 7 to 8, comprising an operative class II DxDD domain.
10. The polypeptide according to any one of the preceding items, further comprising a plastidial targeting signal.
11. A polynucleotide encoding a polypeptide as defined in any one of the preceding items.
12. The polynucleotide according to item 11, wherein the polynucleotide has a sequence with at least 85% identity to a sequence selected from the group consisting of SEQ ID NO: 5 [CfTPS2], SEQ ID NO: 6 [CfTPS3], SEQ ID NO: 7 [CfTPS4] and SEQ ID NO: 8 [EpTPS8].
13. The polynucleotide according to any one of items 11 to 12, wherein the polynucleotide has a sequence with at least 85% identity to SEQ ID NO: 5 [CfTPS2].
14. The polynucleotide according to any one of items 11 to 12, wherein the polynucleotide has a sequence with at least 85% identity to SEQ ID NO: 6 [CfTPS3].
15. The polynucleotide according to any one of items 11 to 12, wherein the polynucleotide has a sequence with at least 85% identity to SEQ ID NO: 7 [CfTPS4].
16. The polynucleotide according to any one of items 11 to 12, wherein the polynucleotide has a sequence with at least 85% identity to SEQ ID NO: 8 [EpTPS8].
17. The polynucleotide according to any one of items 11 to 16, further comprising a sequence coding for a plastidial targeting signal.
18. The polynucleotide according to any one of items 11 to 17, wherein the polynucleotide is codon-optimised for expression in a host cell.
19. The polynucleotide according to item 18, wherein the host cell is selected from the group comprising bacterial cell, yeast cells, fungal cells, plant cells, mammalian cells and insect cells.
20. A vector comprising at least one polynucleotide as defined in any one of items 11 to 19.
21. The vector according to item 20, wherein the vector comprises one polynucleotide as defined in any one of items 14 to 16 and one polynucleotide as defined in item 13.
22. A host cell comprising the polynucleotide according to any one of items 11 to19, and/or the vector according to any one of items 20 to 21.
23. The host cell according to item 22, capable of producing (13R)-manoyl oxide.
24. The cell according any one of items 22 to 23, wherein the cell expresses:
   (i) a first polypeptide having a sequence at least 70% identical to SEQ ID NO: 1 [CfTPS2]; and
   (ii) a second polypeptide having a sequence at least 70% identical to SEQ ID NO: 2 [CfTPS3], SEQ ID NO: 3 [CfTPS4], or SEQ ID NO: 4 [EpTPS8].
25. The cell according to item 24, wherein the first polypeptide has a sequence at least 70% identical to SEQ ID NO: 1 [CfTPS2] and the second polypeptide has a sequence at least 70% identical to SEQ ID NO: 2 [CfTPS3].
26. The cell according to item 24, wherein the first polypeptide has a sequence at least 70% identical to SEQ ID NO: 1 [CfTPS2] and the second polypeptide has a sequence at least 70% identical to SEQ ID NO: 3 [CfTPS4].
27. The cell according to item 24, wherein the first polypeptide has a sequence at least 70% identical to SEQ ID NO: 1 [CfTPS2] and the second polypeptide has a sequence at least 70% identical to SEQ ID NO: 4 [EpTPS8].
28. The cell according to any one of items 22 to 27, wherein the cell is selected from the group comprising bacterial cell, yeast cells, fungal cells, plant cells, mammalian cells and insect cells.
29. The cell according to item 28, wherein the cell is a bacterial cell.
30. The cell according to item 29, wherein the bacteria is *Escherichia coli.*
31. The cell according to item 28, wherein the cell is a yeast cell.
32. The cell according to item 31, wherein the yeast is *Saccharomyces cerevisiae* or *Schizosaccharomyces pombe.*
33. The cell according to any one of items 22 to 32, wherein the cell is capable of secreting (13R)-manoyl oxide.
34. The cell according to any one of items 22 to 33, wherein the cell is transfected with at least one vector for expressing the first and the second polypeptides.
35. The cell according to any one of items 22 to 34, wherein the cell is transfected with at least one vector according to any one of items 20 to 21.
36. The cell according to item 28, wherein the cell is a plant cell selected from the group consisting of *Nicotiana benthamiana* and *Physcomitrella patens.*
37. The cell according to item 36, wherein the cell is a *Nicotiana benthamiana* cell.
38. The cell according to item 37, wherein the *Nicotiana benthamiana* cell is a leaf cell.
39. The cell according to item 38, wherein the *Nicotiana benthamiana* leaf cell is transfected with at least one vector for expressing the first and the second polypeptides.
40. The cell according to item 39, wherein the at least one vector for expressing the first and the second polypeptides is a bacterial vector for transient expression.
41. The cell according to item 40, wherein the bacterial vector is *Agrobacterium tumefaciens.*
42. The cell according to any one of items 40 to 41, wherein the first and second polypeptides are expressed in a transient manner.
43. The cell according to item 42, wherein the vector also encodes a suppressor of gene silencing.
44. The cell according to item 43, wherein the suppressor of gene silencing is the p19 protein of tomato bushy stunt virus.
45. The cell according to any one of items 22 to 44, wherein the cell is further capable of producing GGPP.
46. The cell according to item 45, wherein GGPP is produced via the plastidial methylerythritol 4-phosphate (MEP) pathway.
47. The cell according to any one of items 22 to 46, wherein the cell is further engineered to direct metabolic fluxes.
48. The cell according to any one of items 22 to 47, wherein the (13R)-manoyl oxide is substantially pure.

## Claims

1. An isolated diterpene synthase (diTPS) polypeptide comprising:
i) an amino acid sequence selected from the group consisting of SEQ ID NO: 1 [CfTPS2], SEQ ID NO: 2 [CfTPS3], SEQ ID NO: 3 [CfTPS4] and SEQ ID NO: 4 [EpTPS8];
ii) a biologically active sequence variant of said polypeptide, wherein the sequence variant has at least 75% sequence identity to said SEQ ID NO: 1 [CfTPS2], SEQ ID NO: 2 [CfTPS3], SEQ ID NO: 3 [CfTPS4] or SEQ ID NO: 4 [EpTPS8]; or
iii) a biologically active fragment of at least 50 contiguous amino acids of any of i) through ii), said fragment having at least 75% sequence identity to SEQ ID NO: 1 [CfTPS2], SEQ ID NO: 2 [CfTPS3], SEQ ID NO: 3 [CfTPS4] or SEQ ID NO: 4 [EpTPS8] in a range of overlap of at least 50 amino acids,
wherein the biological activity is diterpene synthase activity.

2. The polypeptide according to claim 1, wherein the polypeptide has a class I diTPS activity, and has a sequence at least 75% identical to, such as at least 80% identical to, such as at least 85% identical to, such as at least 90% identical to, such as at least 95% identical to, such as at least 96% identical to, such as at least 97% identical to, such as at least 98% identical to, such as at least 99% identical to, such as 100% identical to SEQ ID NO: 2 [CfTPS3], SEQ ID NO: 3 [CfTPS4] or SEQ ID NO: 4 [EpTPS8].

3. The polypeptide according to any one of the preceding claims, comprising an operative class I DDxxD domain.

4. The polypeptide according to claim 1, wherein the polypeptide has a class II diTPS activity, and has a sequence at least 75% identical to, such as at least 80% identical to, such as at least 85% identical to, such as at least 90% identical to, such as at least 95% identical to, such as at least 96% identical to, such as at least 97% identical to, such as at least 98% identical to, such as at least 99% identical to, such as 100% identical to SEQ ID NO: 1 [CfTPS2].

5. The polypeptide according to claim 4, comprising an operative class II DxDD domain.

6. The polypeptide according to any one of the preceding claims, further comprising a plastidial targeting signal.

7. A polynucleotide encoding a polypeptide as defined in any one of the preceding claims.

8. The polynucleotide according to claim 7, wherein the polynucleotide has a sequence with at least 85% identity to a sequence selected from the group consisting of SEQ ID NO: 5 [CfTPS2], SEQ ID NO: 6 [CfTPS3], SEQ ID NO: 7 [CfTPS4], SEQ ID NO: 8 [EpTPS8] and SEQ ID NO: 5 [CfTPS2].

9. The polynucleotide according to any one of claims 7 to 8, further comprising a sequence coding for a plastidial targeting signal.

10. The polynucleotide according to any one of claims 7 to 9, wherein the polynucleotide is codon-optimised for expression in a host cell selected from the group comprising bacterial cell, yeast cells, fungal cells, plant cells, mammalian cells and insect cells.

11. A vector comprising at least one polynucleotide as defined in any one of claims 7 to 10.

12. A host cell comprising the polynucleotide according to any one of claims 7 to 10, and/or the vector according to claim 11.

13. The cell according to claim 12, wherein the cell expresses:
(i) a first polypeptide having a sequence at least 70% identical to SEQ ID NO: 1 [CfTPS2]; and
(ii) a second polypeptide having a sequence at least 70% identical to SEQ ID NO: 2 [CfTPS3], SEQ ID NO: 3 [CfTPS4], or SEQ ID NO: 4 [EpTPS8],
wherein the cell is selected from the group comprising bacterial cell, yeast cells, fungal cells, plant cells, mammalian cells and insect cells.

14. The cell according to claim 13, wherein the cell is a *Nicotiana benthamiana* cell transfected with at least one viral vector for transiently expressing the first and the second polypeptides.

15. The cell according to any one of claims 12 to 14, wherein the cell is further naturally capable of producing or further engineered to produce GGPP via the plastidial methylerythritol 4-phosphate (MEP) pathway.


SEQUENCE LISTING

<110>  Technical University of Denmark

<120>  Stereo-specific biosynthesis of (13R) manoyl oxide

<130>  P3428EP00

<160>  61

<170>  PatentIn version 3.5

<210>  1
<211>  773
<212>  PRT
<213>  Unknown

<220>
<223>  TPS2 from Coleus forskohlii

<220>
<221>  CfTPS2
<222>  (1)..(773)
<223>  Coleus forskohlii TPS2

<220>
<221>  DOMAIN
<222>  (358)..(361)
<223>  Class II domain

<400>  1

Met Lys Met Leu Met Ile Lys Ser Gln Phe Arg Val His Ser Ile Val
1               5                   10                  15

Ser Ala Trp Ala Asn Asn Ser Asn Lys Arg Gln Ser Leu Gly His Gln
            20                  25                  30

Ile Arg Arg Lys Gln Arg Ser Gln Val Thr Glu Cys Arg Val Ala Ser
        35                  40                  45

Leu Asp Ala Leu Asn Gly Ile Gln Lys Val Gly Pro Ala Thr Ile Gly
    50                  55                  60

Thr Pro Glu Glu Glu Asn Lys Lys Ile Glu Asp Ser Ile Glu Tyr Val
65                  70                  75                  80

Lys Glu Leu Leu Lys Thr Met Gly Asp Gly Arg Ile Ser Val Ser Pro
                85                  90                  95

Tyr Asp Thr Ala Ile Val Ala Leu Ile Lys Asp Leu Glu Gly Gly Asp
            100                 105                 110

Gly Pro Glu Phe Pro Ser Cys Leu Glu Trp Ile Ala Gln Asn Gln Leu
        115                 120                 125

Ala Asp Gly Ser Trp Gly Asp His Phe Phe Cys Ile Tyr Asp Arg Val
    130                 135                 140

Val Asn Thr Ala Ala Cys Val Val Ala Leu Lys Ser Trp Asn Val His
145                 150                 155                 160

Ala Asp Lys Ile Glu Lys Gly Ala Val Tyr Leu Lys Glu Asn Val His
                165                 170                 175

Lys Leu Lys Asp Gly Lys Ile Glu His Met Pro Ala Gly Phe Glu Phe
            180                 185                 190

Val Val Pro Ala Thr Leu Glu Arg Ala Lys Ala Leu Gly Ile Lys Gly
        195                 200                 205

Leu Pro Tyr Asp Asp Pro Phe Ile Arg Glu Ile Tyr Ser Ala Lys Gln
    210                 215                 220

Thr Arg Leu Thr Lys Ile Pro Lys Gly Met Ile Tyr Glu Ser Pro Thr
225                 230                 235                 240

Ser Leu Leu Tyr Ser Leu Asp Gly Leu Glu Gly Leu Glu Trp Asp Lys
                245                 250                 255

Ile Leu Lys Leu Gln Ser Ala Asp Gly Ser Phe Ile Thr Ser Val Ser
            260                 265                 270

Ser Thr Ala Phe Val Phe Met His Thr Asn Asp Leu Lys Cys His Ala
        275                 280                 285

Phe Ile Lys Asn Ala Leu Thr Asn Cys Asn Gly Gly Val Pro His Thr
    290                 295                 300

Tyr Pro Val Asp Ile Phe Ala Arg Leu Trp Ala Val Asp Arg Leu Gln
305                 310                 315                 320

Arg Leu Gly Ile Ser Arg Phe Phe Glu Pro Glu Ile Lys Tyr Leu Met
                325                 330                 335

Asp His Ile Asn Asn Val Trp Arg Glu Lys Gly Val Phe Ser Ser Arg
            340                 345                 350

His Ser Gln Phe Ala Asp Ile Asp Asp Thr Ser Met Gly Ile Arg Leu
        355                 360                 365

Leu Lys Met His Gly Tyr Asn Val Asn Pro Asn Ala Leu Glu His Phe

370                 375                 380

Lys Gln Lys Asp Gly Lys Phe Thr Cys Tyr Ala Asp Gln His Ile Glu
385                 390                 395                 400

Ser Pro Ser Pro Met Tyr Asn Leu Tyr Arg Ala Ala Gln Leu Arg Phe
                405                 410                 415

Pro Gly Glu Glu Ile Leu Gln Gln Ala Leu Gln Phe Ala Tyr Asn Phe
            420                 425                 430

Leu His Glu Asn Leu Ala Ser Asn His Phe Gln Glu Lys Trp Val Ile
        435                 440                 445

Ser Asp His Leu Ile Asp Glu Val Arg Ile Gly Leu Lys Met Pro Trp
    450                 455                 460

Tyr Ala Thr Leu Pro Arg Val Glu Ala Ser Tyr Tyr Leu Gln His Tyr
465                 470                 475                 480

Gly Gly Ser Ser Asp Val Trp Ile Gly Lys Thr Leu Tyr Arg Met Pro
                485                 490                 495

Glu Ile Ser Asn Asp Thr Tyr Lys Ile Leu Ala Gln Leu Asp Phe Asn
            500                 505                 510

Lys Cys Gln Ala Gln His Gln Leu Glu Trp Met Ser Met Lys Glu Trp
        515                 520                 525

Tyr Gln Ser Asn Asn Val Lys Glu Phe Gly Ile Ser Lys Lys Glu Leu
    530                 535                 540

Leu Leu Ala Tyr Phe Leu Ala Ala Ala Thr Met Phe Glu Pro Glu Arg
545                 550                 555                 560

Thr Gln Glu Arg Ile Met Trp Ala Lys Thr Gln Val Val Ser Arg Met
                565                 570                 575

Ile Thr Ser Phe Leu Asn Lys Glu Asn Thr Met Ser Phe Asp Leu Lys
            580                 585                 590

Ile Ala Leu Leu Thr Gln Pro Gln His Gln Ile Asn Gly Ser Glu Met
        595                 600                 605

Lys Asn Gly Leu Ala Gln Thr Leu Pro Ala Ala Phe Arg Gln Leu Leu
    610                 615                 620

Lys Glu Phe Asp Lys Tyr Thr Arg His Gln Leu Arg Asn Thr Trp Asn
625                 630                 635                 640

Lys Trp Leu Met Lys Leu Lys Gln Gly Asp Asp Asn Gly Gly Ala Asp
                645                 650                 655

Ala Glu Leu Leu Ala Asn Thr Leu Asn Ile Cys Ala Gly His Asn Glu
            660                 665                 670

Asp Ile Leu Ser His Tyr Glu Tyr Thr Ala Leu Ser Ser Leu Thr Asn
        675                 680                 685

Lys Ile Cys Gln Arg Leu Ser Gln Ile Gln Asp Lys Lys Met Leu Glu
    690                 695                 700

Ile Glu Glu Gly Ser Ile Lys Asp Lys Glu Met Glu Leu Glu Ile Gln
705                 710                 715                 720

Thr Leu Val Lys Leu Val Leu Gln Glu Thr Ser Gly Gly Ile Asp Arg
                725                 730                 735

Asn Ile Lys Gln Thr Phe Leu Ser Val Phe Lys Thr Phe Tyr Tyr Arg
            740                 745                 750

Ala Tyr His Asp Ala Lys Thr Ile Asp Ala His Ile Phe Gln Val Leu
        755                 760                 765

Phe Glu Pro Val Val
    770

<210>  2
<211>  598
<212>  PRT
<213>  Unknown

<220>
<223>  Coleus forskohlii sequence

<220>
<221>  CfTPS3
<222>  (1)..(598)

<220>
<221>  DOMAIN
<222>  (330)..(334)
<223>  class I domain

<400>  2

Met Ser Ser Leu Ala Gly Asn Leu Arg Val Ile Pro Phe Ser Gly Asn
1               5                   10                  15

Arg Val Gln Thr Arg Thr Gly Ile Leu Pro Val His Gln Thr Pro Met
            20                  25                  30

Ile Thr Ser Lys Ser Ser Ala Ala Val Lys Cys Ser Leu Thr Thr Pro
        35                  40                  45

Thr Asp Leu Met Gly Lys Ile Lys Glu Val Phe Asn Arg Glu Val Asp
    50                  55                  60

Thr Ser Pro Ala Ala Met Thr Thr His Ser Thr Asp Ile Pro Ser Asn
65                  70                  75                  80

Leu Cys Ile Ile Asp Thr Leu Gln Arg Leu Gly Ile Asp Gln Tyr Phe
                85                  90                  95

Gln Ser Glu Ile Asp Ala Val Leu His Asp Thr Tyr Arg Leu Trp Gln
            100                 105                 110

Leu Lys Lys Lys Asp Ile Phe Ser Asp Ile Thr Thr His Ala Met Ala
        115                 120                 125

Phe Arg Leu Leu Arg Val Lys Gly Tyr Glu Val Ala Ser Asp Glu Leu
    130                 135                 140

Ala Pro Tyr Ala Asp Gln Glu Arg Ile Asn Leu Gln Thr Ile Asp Val
145                 150                 155                 160

Pro Thr Val Val Glu Leu Tyr Arg Ala Ala Gln Glu Arg Leu Thr Glu
                165                 170                 175

Glu Asp Ser Thr Leu Glu Lys Leu Tyr Val Trp Thr Ser Ala Phe Leu
            180                 185                 190

Lys Gln Gln Leu Leu Thr Asp Ala Ile Pro Asp Lys Lys Leu His Lys
        195                 200                 205

Gln Val Glu Tyr Tyr Leu Lys Asn Tyr His Gly Ile Leu Asp Arg Met
    210                 215                 220

Gly Val Arg Arg Asn Leu Asp Leu Tyr Asp Ile Ser His Tyr Lys Ser
225                 230                 235                 240

Leu Lys Ala Ala His Arg Phe Tyr Asn Leu Ser Asn Glu Asp Ile Leu
                245                 250                 255

Ala Phe Ala Arg Gln Asp Phe Asn Ile Ser Gln Ala Gln His Gln Lys
            260                 265                 270

Glu Leu Gln Gln Leu Gln Arg Trp Tyr Ala Asp Cys Arg Leu Asp Thr
        275                 280                 285

Leu Lys Phe Gly Arg Asp Val Val Arg Ile Gly Asn Phe Leu Thr Ser
    290                 295                 300

Ala Met Ile Gly Asp Pro Glu Leu Ser Asp Leu Arg Leu Ala Phe Ala
305                 310                 315                 320

Lys His Ile Val Leu Val Thr Arg Ile Asp Asp Phe Phe Asp His Gly
                325                 330                 335

Gly Pro Lys Glu Glu Ser Tyr Glu Ile Leu Glu Leu Val Lys Glu Trp
            340                 345                 350

Lys Glu Lys Pro Ala Gly Glu Tyr Val Ser Glu Glu Val Glu Ile Leu
        355                 360                 365

Phe Thr Ala Val Tyr Asn Thr Val Asn Glu Leu Ala Glu Met Ala His
    370                 375                 380

Ile Glu Gln Gly Arg Ser Val Lys Asp Leu Leu Val Lys Leu Trp Val
385                 390                 395                 400

Glu Ile Leu Ser Val Phe Arg Ile Glu Leu Asp Thr Trp Thr Asn Asp
                405                 410                 415

Thr Ala Leu Thr Leu Glu Glu Tyr Leu Ser Gln Ser Trp Val Ser Ile
            420                 425                 430

Gly Cys Arg Ile Cys Ile Leu Ile Ser Met Gln Phe Gln Gly Val Lys
        435                 440                 445

Leu Ser Asp Glu Met Leu Gln Ser Glu Glu Cys Thr Asp Leu Cys Arg
    450                 455                 460

Tyr Val Ser Met Val Asp Arg Leu Leu Asn Asp Val Gln Thr Phe Glu
465                 470                 475                 480

Lys Glu Arg Lys Glu Asn Thr Gly Asn Ser Val Ser Leu Leu Gln Ala
                485                 490                 495

Ala His Lys Asp Glu Arg Val Ile Asn Glu Glu Glu Ala Cys Ile Lys
            500                 505                 510

Val Lys Glu Leu Ala Glu Tyr Asn Arg Arg Lys Leu Met Gln Ile Val

515                 520                 525

Tyr Lys Thr Gly Thr Ile Phe Pro Arg Lys Cys Lys Asp Leu Phe Leu
    530                 535                 540

Lys Ala Cys Arg Ile Gly Cys Tyr Leu Tyr Ser Ser Gly Asp Glu Phe
545                 550                 555                 560

Thr Ser Pro Gln Gln Met Met Glu Asp Met Lys Ser Leu Val Tyr Glu
                565                 570                 575

Pro Leu Pro Ile Ser Pro Pro Glu Ala Asn Asn Ala Ser Gly Glu Lys
            580                 585                 590

Met Ser Cys Val Ser Asn
        595

<210>  3
<211>  587
<212>  PRT
<213>  Unknown

<220>
<223>  CfTPS4 from Coleus forskohlii

<220>
<221>  CfTPS4
<222>  (1)..(587)

<220>
<221>  DOMAIN
<222>  (331)..(335)
<223>  Class I domain

<400>  3

Met Ser Ile Thr Ile Asn Leu Arg Val Ile Ala Phe Pro Gly His Gly
1               5                   10                  15

Val Gln Ser Arg Gln Gly Ile Phe Ala Val Met Glu Phe Pro Arg Asn
            20                  25                  30

Lys Asn Thr Phe Lys Ser Ser Phe Ala Val Lys Cys Ser Leu Ser Thr
        35                  40                  45

Pro Thr Asp Leu Met Gly Lys Ile Lys Glu Lys Leu Arg Glu Lys Val
    50                  55                  60

Asp Asn Ser Gly Ala Ala Met Ala Thr Asp Ser Ala Asp Met Pro Thr
65                  70                  75                  80

Asn Leu Cys Ile Val Asp Ser Leu Gln Arg Leu Gly Val Glu Lys Tyr
                85                  90                  95

Phe Gln Ser Glu Ile Asp Thr Val Leu Asp Asp Ala Tyr Arg Leu Trp
            100                 105                 110

Gln Leu Lys Gln Lys Asp Ile Phe Ser Asp Ile Thr Thr His Ala Met
        115                 120                 125

Ala Phe Arg Leu Leu Arg Val Lys Gly Tyr Asp Val Ser Ser Glu Glu
    130                 135                 140

Leu Ala Pro Tyr Ala Asp Gln Glu Gly Met Asn Leu Gln Thr Ile Asp
145                 150                 155                 160

Leu Ala Ala Val Ile Glu Leu Tyr Arg Ala Ala Gln Glu Arg Val Ala
                165                 170                 175

Glu Glu Asp Ser Thr Leu Glu Lys Leu Tyr Val Trp Thr Ser Thr Phe
            180                 185                 190

Leu Lys Gln Gln Leu Leu Ala Gly Ala Ile Pro Asp Gln Lys Leu His
        195                 200                 205

Lys Gln Val Glu Tyr Tyr Leu Lys Asn Tyr His Gly Ile Leu Asp Arg
    210                 215                 220

Met Gly Val Arg Lys Gly Leu Asp Leu Tyr Asp Ala Gly Tyr Tyr Lys
225                 230                 235                 240

Ala Leu Lys Ala Ala Asp Arg Leu Val Asp Leu Cys Asn Glu Asp Leu
                245                 250                 255

Leu Ala Phe Ala Arg Gln Asp Phe Asn Ile Asn Gln Ala Gln His Arg
            260                 265                 270

Lys Glu Leu Glu Gln Leu Gln Arg Trp Tyr Ala Asp Cys Arg Leu Asp
        275                 280                 285

Lys Leu Glu Phe Gly Arg Asp Val Val Arg Val Ser Asn Phe Leu Thr
    290                 295                 300

Ser Ala Ile Leu Gly Asp Pro Glu Leu Ser Glu Val Arg Leu Val Phe
305                 310                 315                 320

Ala Lys His Ile Val Leu Val Thr Arg Ile Asp Asp Phe Phe Asp His
                325                 330                 335

Gly Gly Pro Arg Glu Glu Ser His Lys Ile Leu Glu Leu Ile Lys Glu
        340                 345                 350

Trp Lys Glu Lys Pro Ala Gly Glu Tyr Val Ser Lys Glu Val Glu Ile
    355                 360                 365

Leu Tyr Thr Ala Val Tyr Asn Thr Val Asn Glu Leu Ala Glu Arg Ala
    370                 375                 380

Asn Val Glu Gln Gly Arg Asn Val Glu Pro Phe Leu Arg Thr Leu Trp
385                 390                 395                 400

Val Gln Ile Leu Ser Ile Phe Lys Ile Glu Leu Asp Thr Trp Ser Asp
                405                 410                 415

Asp Thr Ala Leu Thr Leu Asp Asp Tyr Leu Asn Asn Ser Trp Val Ser
            420                 425                 430

Ile Gly Cys Arg Ile Cys Ile Leu Met Ser Met Gln Phe Ile Gly Met
        435                 440                 445

Lys Leu Pro Glu Glu Met Leu Leu Ser Glu Glu Cys Val Asp Leu Cys
    450                 455                 460

Arg His Val Ser Met Val Asp Arg Leu Leu Asn Asp Val Gln Thr Phe
465                 470                 475                 480

Glu Lys Glu Arg Lys Glu Asn Thr Gly Asn Ala Val Ser Leu Leu Leu
                485                 490                 495

Ala Ala His Lys Gly Glu Arg Ala Phe Ser Glu Glu Glu Ala Ile Ala
            500                 505                 510

Lys Ala Lys Tyr Leu Ala Asp Cys Asn Arg Arg Ser Leu Met Gln Ile
        515                 520                 525

Val Tyr Lys Thr Gly Thr Ile Phe Pro Arg Lys Cys Lys Asp Met Phe
    530                 535                 540

Leu Lys Val Cys Arg Ile Gly Cys Tyr Leu Tyr Ala Ser Gly Asp Glu
545                 550                 555                 560

Phe Thr Ser Pro Gln Gln Met Met Glu Asp Met Lys Ser Leu Val Tyr
                565                 570                 575

Glu Pro Leu Gln Ile His Pro Pro Pro Ala Asn
            580                 585

<210>  4
<211>  792
<212>  PRT
<213>  Euphorbia peplus

<220>
<221>  EpTPS8
<222>  (1)..(792)

<220>
<221>  DOMAIN
<222>  (540)..(544)
<223>  Class I domain

<400>  4

Met Gln Val Ser Leu Ser Leu Thr Thr Gly Ser Glu Pro Cys Ile Thr
1               5                   10                  15

Arg Ile His Ala Pro Ser Asp Ala Pro Leu Lys Gln Arg Asn Asn Glu
            20                  25                  30

Arg Glu Lys Gly Thr Leu Glu Leu Asn Gly Lys Val Ser Leu Lys Lys
        35                  40                  45

Met Gly Glu Met Leu Arg Thr Ile Glu Asn Val Pro Ile Val Gly Ser
    50                  55                  60

Thr Ser Ser Tyr Asp Thr Ala Trp Val Gly Met Val Pro Cys Ser Ser
65                  70                  75                  80

Asn Ser Ser Lys Pro Leu Phe Pro Glu Ser Leu Lys Trp Ile Met Glu
                85                  90                  95

Asn Gln Asn Pro Glu Gly Asn Trp Ala Val Asp His Ala His His Pro
            100                 105                 110

Leu Leu Leu Lys Asp Ser Leu Ser Ser Thr Leu Ala Cys Val Leu Ala
        115                 120                 125

Leu His Lys Trp Asn Leu Ala Pro Gln Leu Val His Ser Gly Leu Asp
    130                 135                 140

Phe Ile Gly Ser Asn Leu Trp Ala Ala Met Asp Phe Arg Gln Arg Ser
145                 150                 155                 160

Pro Leu Gly Phe Asp Val Ile Phe Pro Gly Met Ile His Gln Ala Ile
                165                 170                 175

Asp Leu Gly Ile Asn Leu Pro Phe Asn Asn Ser Ser Ile Glu Asn Met
            180                 185                 190

Leu Thr Asn Pro Leu Leu Asp Ile Gln Ser Phe Glu Ala Gly Lys Thr
        195                 200                 205

Ser His Ile Ala Tyr Phe Ala Glu Gly Leu Gly Ser Arg Leu Lys Asp
    210                 215                 220

Trp Glu Gln Leu Leu Gln Tyr Gln Thr Ser Asn Gly Ser Leu Phe Asn
225                 230                 235                 240

Ser Pro Ser Thr Thr Ala Ala Ala Ala Ile His Leu Arg Asp Glu Lys
                245                 250                 255

Cys Leu Asn Tyr Leu His Ser Leu Thr Lys Gln Phe Asp Asn Gly Ala
            260                 265                 270

Val Pro Thr Leu Tyr Pro Leu Asp Ala Arg Thr Arg Ile Ser Ile Ile
        275                 280                 285

Asp Ser Leu Glu Lys Phe Gly Ile His Ser His Phe Ile Gln Glu Met
    290                 295                 300

Thr Ile Leu Leu Asp Gln Ile Tyr Ser Phe Trp Lys Glu Gly Asn Glu
305                 310                 315                 320

Glu Ile Phe Lys Asp Pro Gly Cys Cys Ala Thr Ala Phe Arg Leu Leu
                325                 330                 335

Arg Lys His Gly Tyr Asp Val Ser Ser Asp Ser Leu Ala Glu Phe Glu
            340                 345                 350

Lys Lys Glu Ile Phe Tyr His Ser Ser Ala Ala Ser Ala His Glu Ile
        355                 360                 365

Asp Thr Lys Ser Ile Leu Glu Leu Phe Arg Ala Ser Gln Met Lys Ile
    370                 375                 380

Leu Gln Asn Glu Pro Ile Leu Asp Arg Ile Tyr Asp Trp Thr Ser Ile
385                 390                 395                 400

Phe Leu Arg Asp Gln Leu Val Lys Gly Leu Ile Glu Asn Lys Ser Leu
                405                 410                 415

Tyr Glu Glu Val Asn Phe Ala Leu Gly His Pro Phe Ala Asn Leu Asp
            420                 425                 430

Arg Leu Glu Ala Arg Ser Tyr Ile Asp Asn Tyr Asp Pro Tyr Asp Val
        435                 440                 445

Pro Leu Leu Lys Thr Ser Tyr Arg Ser Ser Asn Ile Asp Asn Lys Asp
    450                 455                 460

Leu Trp Thr Ile Ala Phe Gln Asp Phe Asn Lys Cys Gln Ala Leu His
465                 470                 475                 480

Arg Val Glu Leu Asp Tyr Leu Glu Lys Trp Val Lys Glu Tyr Lys Leu
                485                 490                 495

Asp Thr Leu Lys Trp Ala Arg Gln Lys Thr Glu Tyr Ala Leu Phe Thr
            500                 505                 510

Ile Gly Ala Ile Leu Ser Glu Pro Glu Tyr Ala Asp Ala Arg Ile Ser
        515                 520                 525

Trp Ser Gln Asn Thr Val Phe Val Thr Ile Val Asp Asp Phe Phe Asp
    530                 535                 540

Tyr Gly Gly Ser Leu Asp Glu Cys Arg Asn Leu Ile Asn Leu Met His
545                 550                 555                 560

Lys Trp Asp Asp His Leu Thr Val Gly Phe Leu Ser Glu Lys Val Glu
                565                 570                 575

Ile Val Phe Tyr Ser Met Tyr Gly Thr Leu Asn Asp Leu Ala Ala Lys
            580                 585                 590

Ala Glu Val Arg Gln Gly Arg Cys Val Arg Ser His Leu Val Asn Leu
        595                 600                 605

Trp Ile Trp Val Met Glu Asn Met Leu Lys Glu Arg Glu Trp Ala Asp
    610                 615                 620

Tyr Asn Leu Val Pro Thr Phe Tyr Glu Tyr Val Ala Ala Gly His Ile
625                 630                 635                 640

Thr Ile Gly Leu Gly Pro Val Leu Leu Ile Ala Leu Tyr Phe Met Gly
                645                 650                 655

Tyr Pro Leu Ser Glu Asp Val Val Gln Ser Gln Glu Tyr Lys Gly Val
            660                 665                 670

Tyr Leu Asn Val Ser Ile Ile Ala Arg Leu Leu Asn Asp Arg Val Thr
        675                 680                 685

Val Lys Arg Glu Ser Ala Gln Gly Lys Leu Asn Gly Val Ser Leu Phe
    690                 695                 700

Val Glu His Gly Arg Gly Ala Val Asp Glu Glu Thr Ser Met Lys Glu
705                 710                 715                 720

Val Glu Arg Leu Val Glu Ser His Lys Arg Glu Leu Leu Arg Leu Ile
                725                 730                 735

Val Gln Lys Thr Glu Gly Ser Val Val Pro Gln Ser Cys Lys Asp Leu
            740                 745                 750

Ala Trp Arg Val Ser Lys Val Leu His Leu Leu Tyr Met Asp Asp Asp
        755                 760                 765

Gly Phe Thr Cys Pro Val Lys Met Leu Asn Ala Thr Asn Ala Ile Val
    770                 775                 780

Asn Glu Pro Leu Leu Leu Thr Ser
785                 790

<210>  5
<211>  2322
<212>  DNA
<213>  Unknown

<220>
<223>  CfTPS2 from Coleus forskholii

<220>
<221>  CfTPS2
<222>  (1)..(2322)

<400>  5
atgaaaatgt tgatgatcaa aagtcaattt cgtgtacatt caatagtcag tgcatgggcg      60

aacaacagca ataaaaggca gtcattgggt caccaaattc gtcgaaagca aagatcacaa     120

gtaaccgagt gtcgagttgc aagtctggat gcgttgaatg gaattcaaaa agtcggccca     180

gccaccattg ggactcctga agaggaaaat aaaaagattg aggattccat tgagtacgtg     240

aaggagttgt tgaagacaat gggcgacggg cgaatcagcg tttccccgta cgacacagca     300

atagttgccc tgattaagga cttggaagga ggtgatggac cagagtttcc atcttgtcta     360

gagtggattg cacagaatca actggctgat ggttcttggg gggatcactt cttctgtatt     420

tatgatcggg ttgttaatac agcagcttgt gtggtcgcct taaagtcgtg gaacgttcac     480

gcagacaaga ttgagaaagg agcagtgtac ctgaaggaga atgtgcataa acttaaagat     540

gggaagattg agcacatgcc cgcagggttt gaatttgtgg ttcctgccac tcttgaaaga     600

gccaaagcct tggggatcaa aggtcttccc tatgatgatc ctttcatcag ggaaatttat 660

agtgcaaaac aaacaagatt gaccaagata ccaaagggca tgatctacga atctccaact 720

tctttattat atagtttaga cggtctggaa ggcttggagt gggacaagat actgaaactg 780

cagtcggccg atggctcatt catcacctct gtgtcgtcta ctgccttcgt attcatgcac 840

accaacgacc ttaaatgcca cgccttcatc aaaaatgccc tcaccaattg caacggggga 900

gtaccccaca cgtatccagt ggatatcttc gcacgacttt gggcagtgga ccgactgcaa 960

cgcctcggaa tatctcgatt ctttgagcct gagatcaaat atttaatgga tcacatcaat 1020

aacgtgtgga gggagaaggg agttttcagt tcaaggcatt cacaatttgc ggatattgac 1080

gacacatcca tgggcatcag gcttctgaaa atgcacggat acaatgtcaa cccaaatgca 1140

cttgaacatt tcaaacagaa agatgggaag tttacatgct atgctgatca acatatcgag 1200

tctccatccc ccatgtacaa tctctacagg gctgctcagc ttcgttttcc aggagaagaa 1260

attcttcaac aagcccttca atttgcctat aattttctac atgaaaacct agccagcaat 1320

cactttcaag aaaaatgggt catatccgac cacctaattg atgaggtaag gatcgggctg 1380

aagatgccat ggtacgccac cctaccgcga gtggaggctt catactatct tcaacattat 1440

ggtggatcca gcgacgtatg gattggcaaa actttataca gaatgccaga aatcagtaac 1500

gacacataca aaatacttgc acaattggac ttcaacaaat gtcaagcaca acatcagttg 1560

gaatggatgt ccatgaaaga gtggtatcaa agtaataatg ttaaagaatt tgggataagc 1620

aagaaagaac ttcttcttgc ttactttttg gctgctgcaa ccatgtttga acccgaacgc 1680

acacaagaga ggattatgtg ggcgaaaact caagtcgttt ctcggatgat cacatcattt 1740

ctcaacaaag aaaacacaat gtcattcgac ctaaagattg cacttttaac ccaaccccaa 1800

catcaaataa atggttctga gatgaagaat ggacttgctc aaactcttcc tgcagccttc 1860

cgacaactac tcaaggaatt cgacaaatac acaagacatc aattgaggaa tacttggaac 1920

aaatggttga tgaaactgaa gcaaggagat gacaatggcg gcgcagatgc agagctcctt 1980

gcaaacacat taaacatatg tgctggacat aacgaagaca tattatcgca ctatgaatac 2040

accgctctct cctccctcac aaacaaaata tgtcagcgtc taagtcaaat tcaagataaa 2100

aagatgctgg aaattgagga ggggagcata aaagataagg agatggagct cgaaatacaa 2160

acattggtga agttagtcct ccaagaaacc agtgggggta tcgatagaaa catcaagcaa 2220

acatttttat cagtattcaa gacattttac tacagggcct accacgatgc taagactatc 2280

gatgcccata ttttccaagt actatttgaa ccagtggtct ga 2322

<210> 6
<211> 1704
<212> DNA

<213>  Unknown

<220>
<223>  CfTPS3 from Coleus forskohlii


<220>
<221>  CfTPS3
<222>  (1)..(1704)

<400>  6
atgatcacct ctaaatcatc tgcagctgtt aaatgcagcc tcaccacgcc aacagatttg      60

atggggaaaa taaaagaggt cttcaacagg gaagtcgata cttctccggc agccatgact     120

actcattcta cagatatacc ctctaatctc tgcataatcg acaccctcca gaggctggga     180

atcgaccaat acttccaatc cgaaatcgac gctgttctac atgatacata caggttatgg     240

caactgaaaa agaaagatat attttcggat attactactc atgcaatggc gttcagactt     300

ttgcgagtca aaggatatga agttgcatca gacgaactgg ctccatacgc tgatcaagag     360

cgcattaacc tgcaaaccat tgatgtgccg acagttgttg agctatacag agcagcacag     420

gagagattaa ctgaagaaga tagcactctt gagaaactgt atgtttggac cagcgccttt     480

ctgaagcagc agttgctcac tgatgccatt cctgacaaga aattgcacaa acaagtggaa     540

tactacttga agaactacca tggcatatta gatagaatgg gagtgagacg aaacctcgac     600

ctatatgaca taagccatta taaaagtctc aaagctgctc acaggttcta taatctgagt     660

aatgaagata tcctagcatt tgcgaggcaa gattttaata ttagccaagc ccaacaccag     720

aaagaacttc agcagctgca aaggtggtat gcagattgta ggttggacac gttgaaattt     780

ggaagagatg tagtgcgtat aggaaatttt ctgacttcag caatgattgg tgatcctgaa     840

ttgtctgacc tccgtctagc gtttgccaaa catatagtgc tcgtaacacg tattgatgat     900

tttttcgatc acggtgggcc taaagaagaa tcatacgaga tccttgaatt agtaaaagaa     960

tggaaagaga agccagcagg agaatatgtt tctgaagaag ttgaaatcct atttacagca    1020

gtatacaata cagtaaacga gttggcagaa atggctcata tcgaacaagg acgaagcgtt    1080

aaagaccttc tagttaaact gtgggttgaa atactatcag ttttcagaat agaattggat    1140

acatggacca acgacacagc acttacctta gaagagtact tgtcacaatc ctgggtgtcc    1200

attggctgca gaatctgcat tctcatatca atgcaattcc aaggtgtaaa attatctgat    1260

gaaatgcttc agagtgaaga atgcactgat ttgtgtcggt atgtttcaat ggttgaccgg    1320

ctgctcaacg atgtgcaaac ttttgagaag gaacgcaagg aaaatacagg aaatagtgtg    1380

agccttctgc aagcagctca caaagatgaa agagtcatta atgaagagga agcttgtata    1440

aaggtaaaag aattggctga atataacagg agaaaactga tgcagattgt ctacaaaaca    1500

ggaaccattt tcccaagaaa atgcaaagat ctgtttttga aggcatgcag aattggttgt    1560

tatttgtact caagtggcga cgaatttact tcgcctcaac aaatgatgga agatatgaag 1620

tcactggttt atgaacccct accaatttct cctcctgaag ctaataatgc aagtggagaa 1680

aaaatgagtt gtgtcagcaa ctag 1704

<210> 7
<211> 1740
<212> DNA
<213> Unknown

<220>
<223> CfTPS4 from Coleus forskohlii

<220>
<221> CfTPS4
<222> (1)..(1740)

<400> 7
atgtcaatca ccatcaacct tcgagttatc gctttccccg gccatggagt tcagagcagg 60

caaggaatat ttgcagtcat ggaatttcca aggaacaaga acacctttaa atcatccttt 120

gctgttaaat gcagcctctc tactccaaca gatttgatgg gaaagataaa agaaaagttg 180

agcgagaagg ttgataattc tgtggcagcc atggctactg attctgccga tatgcccact 240

aatctctgca tcgtcgactc cctccagagg ctgggagtcg aaaaatattt ccaatccgaa 300

atcgacactg ttctcgatga tgcataccgg ttatggcagc tgaagcagaa agatatattt 360

tcagacatta ctactcatgc aatggcgttt agacttctgc gagtcaaagg atacgatgtt 420

tcatcagagg agctggctcc atacgctgat caagagggca tgaacttgca aacgattgat 480

ctggcggcgg tcatcgagct gtacagagca gcacaggaga gagtggctga ggaagacagc 540

actcttgaga aactgtatgt ctggaccagc acctttctga agcagcagtt gctggctggc 600

gccattcctg accagaaatt gcacaaacag gtggagtact acttgaagaa ctaccacggc 660

atattagata gaatgggagt tagaaaagga ctcgacctgt atgatgctgg ctattacaag 720

gccctcaaag ctgcagatag gttggttgat ctatgcaatg aagaccttct agcatttgca 780

aggcaagatt ttaatattaa ccaagcccaa caccgcaaag aacttgagca actgcaaagg 840

tggtatgcag attgtaggtt ggacaaactc gagtttggaa gagatgtggt gcgtgtatcg 900

aattttctga cttcagccat ccttggtgat ccagagcttt ctgaagtccg tctagtgttt 960

gccaaacata ttgtgctagt gactaggata gatgattttt tcgatcatgg cgggcctaga 1020

gaagaatcac acaagatcct tgaactaata aaagaatgga aagagaagcc agctggagaa 1080

tatgtttcca agaagttgga gatcctatat accgcggtgt acaatacggt aaacgagttg 1140

gcagagaggg caaatgttga acaaggcgaa aatgttgaac catttctacg tacactgtgg 1200

gttcaaatac tgtcgatttt caagatagag ttggatacat ggagcgatga cacagcacta 1260

accttggatg attacttgaa caactcatgg gtgtcgattg gttgtagaat ctgcattctc 1320

atgtccatgc aattcattgg tatgaagtta ccagaagaaa tgcttctcag tgaagagtgc 1380

gttgatttgt gtaggcatgt ttccatggtc gaccgtctgc tcaatgatgt ccaaactttt 1440

gagaaggaac ggaaagaaaa tacaggaaac gctgtgagcc ttctgctagc agctcacaag 1500

ggtgaaagag ccttcagtga agaggaagcc atagcaaaag cgaaatattt ggctgactgt 1560

aacaggagaa gtctgatgca gattgtgtat aaaacaggaa ccattttccc aagaaaatgc 1620

aaagatatgt tcttgaaggt gtgcaggatt ggttgctatt tgtatgcgag tggcgacgaa 1680

tttacttccc ctcaacaaat gatggaagat atgaagtcat tagtttatga gcccctccaa 1740

<210> 8
<211> 2379
<212> DNA
<213> Euphorbia peplus

<220>
<221> EpTPS8
<222> (1)..(2379)

<400> 8
atgcaagtct ctctctccct caccactggc tccgagcctt gcattaccag aatccatgct 60

ccatctgatg caccattgaa acagcggaac aatgaaagag agaaggggac actagaacta 120

aatgggaaag tttcgctgaa gaaaatggga gagatgctga ggacaataga aaatgtacct 180

atagtaggta gtacgtcgtc gtatgataca gcatgggtcg gtatggtgcc ttgttcatcg 240

aattcgtcga aaccgctatt tccagaaagc ttgaaatgga taatggagaa tcaaaatcca 300

gaagggaatt gggcagttga tcatgctcat catcctcttc ttcttaaaga ctctctttct 360

tccactcttg cttgtgtcct tgctttgcac aaatggaatt tggctcccca gcttgttcac 420

tccggtttgg acttcatcgg ctctaatcta tgggcagcta tggactttcg acaacgatct 480

cctctcggat ttgatgttat atttccagga atgatccacc aagctattga tttgggcatc 540

aatcttcctt tcaacaactc ttcaattgag aacatgctca ctaacccact tttggacatt 600

caaagttttg aagcaggaaa aactagccat attgcatact ttgccgaggg attaggaagt 660

agattaaagg attgggaaca acttcttcaa tatcaaacaa gtaacggttc gctcttcaat 720

tcaccttcta caactgctgc cgctgctatt catctacgtg acgaaaaatg tcttaattac 780

ttgcattctc taaccaaaca attcgataat ggtgctgttc caacacttta tcctctcgat 840

gcgcgtacca gaatctccat aatcgatagt ttggaaaagt ttggatccca ttcacatttc 900

atccaagaaa tgacaattct actagatcaa atatacagct tctggaaaga agggaatgaa 960

gaaatattta aagaccctgg atgttgtgca acagcattcc gactgctgcg aaagcatggt 1020

tatgatgttt cttcagattc cttggcagag tttgagaaaa aagagatatt ttaccattca 1080

tcagcagcta gtgcacacga aatcgatacc aagtctattc tagaattatt cagagcttcc 1140

caaatgaaaa ttttgcaaaa tgaaccaata ctcgacagaa tttacgattg gactagcatt 1200

tttctgagag accagctagt gaaaggtcta atcgaaaaca agagtctgta cgaagaagtt 1260

aattttgctt tgggacatcc atttgctaat ctggatagac tcgaagctcg ttcttacatc 1320

gacaattacg atccatatga tgtcccactt cttaagacat cttacaggtc atccaatatt 1380

gataacaaag atctatggac aattgcattc caagatttca acaagtgcca agccttgcac 1440

cgtgtggaac ttgattatct ggagaaatgg gtgaaagaat acaaattgga cactctgaag 1500

tgggcaaggc agaagactga gtatgcatta tttacgatag gcgcaatcct ttcggagcct 1560

gaatacgctg atgctcgcat ctcatggtca cagaatactg tttttgtgac tattgttgat 1620

gatttctttg actatggtgg ttcgttggat gaatgtcgta acctcattaa ccttatgcac 1680

aagtgggatg atcacttaac cgttggattc ttgtcggaaa aagtggaaat cgtattttat 1740

tcgatgtatg gcacactcaa tgaccttgct gccaaagccg aagtacgaca aggccgatgt 1800

gttcgaagtc acttagttaa tttatggatc tgggtgatgg aaaacatgtt aaaggagaga 1860

gaatgggcag attacaatct ggtgcctaca ttttacgagt acgtagccgc tggacatata 1920

actatcggct taggacctgt gcttcttatt gccctctatt ttatggggta tccgctttct 1980

gaggatgtgg ttcaaagtca agaatacaag ggtgtttatt tgaatgtcag catcattgct 2040

cgacttctaa atgatcgcgt aactgttaag agggaaagtg cgcaaggaaa gcttaatggt 2100

gtgtcattgt tcgtcgaaca tggtcgtggc gcggttgatg aggaaactag tatgaaggaa 2160

gtagaaagac tggtagagag ccataagaga gaattattaa gattgattgt gcagaaaacg 2220

gaaggcagtg tcgtcccgca aagttgcaaa gatctagctt ggagggttag caaagttttg 2280

caccttctat atatggatga tgatggtttt acatgtcctg tgaagatgct taatgctaca 2340

aatgcaattg tcaacgaacc actcctttta acttcataa 2379

<210> 9
<211> 4
<212> PRT
<213> Unknown

<220>
<223> Class II diterpene synthase domain


<220>
<221> DOMAIN
<222> (1)..(4)
<223> X: any amino acid

<400> 9

Asp Xaa Asp Asp

1

<210>  10
<211>  5
<212>  PRT
<213>  Unknown

<220>
<223>  Class I diterpene synthase domain

<220>
<221>  DOMAIN
<222>  (1)..(5)
<223>  X: any amino acid

<400>  10

Asp Asp Xaa Xaa Asp
1               5

<210>  11
<211>  28
<212>  DNA
<213>  Artificial sequence

<220>
<223>  Bri046 forward primer for amplification of CfTPS1

<220>
<221>  primer_bind
<222>  (1)..(28)
<223>  CfTPS1_F

<400>  11
cagaatgggg tctctatcca ctatgaac                                          28

<210>  12
<211>  22
<212>  DNA
<213>  Artificial sequence

<220>
<223>  Bri047 reverse primer for amplification of CfTPS1

<220>
<221>  primer_bind
<222>  (1)..(22)
<223>  CfTPS1_R

<400>  12
cagcatattc aggcgactgg tt                                                22

<210>  13
<211>  30
<212>  DNA
<213>  Artificial sequence

<220>
<223>  Bri048 forward primer for amplification of CfTPS2


<220>
<221>  primer_bind
<222>  (1)..(30)
<223>  CfTPS2_F

<400>  13
agattgagga ttccattgag tacgtgaagg                                        30


<210>  14
<211>  30
<212>  DNA
<213>  Artificial sequence

<220>
<223>  Bri049 reverse primer for amplification of CfTPS2


<220>
<221>  primer_bind
<222>  (1)..(30)
<223>  CfTPS2_R

<400>  14
gaagtttaat atccttcatt ctttattaca                                        30


<210>  15
<211>  27
<212>  DNA
<213>  Artificial sequence

<220>
<223>  Bri099 forward primer for amplification of CfTPS3


<220>
<221>  primer_bind
<222>  (1)..(27)
<223>  CfTPS3_F

<400>  15
agctccattc aactagagtc atgtcgt                                           27


<210>  16
<211>  28
<212>  DNA
<213>  Artificial sequence

<220>
<223>  Bri100 reverse primer for amplification of CfTPS3


<220>
<221>  primer_bind
<222>  (1)..(28)
<223>  CfTPS3_R

<400>  16
ttcatctggc ttaactagtt gctgacac                                          28


<210>  17
<211>  25
<212>  DNA
<213>  Artificial sequence

<220>
<223>  Bri0101 forward primer for amplification of CfTPS4


<220>
<221>  primer_bind
<222>  (1)..(25)
<223>  CfTPS4_F

<400>  17
gtgcactctc caccaacgat aaact                                             25


<210>  18
<211>  25
<212>  DNA
<213>  Artificial sequence

<220>
<223>  Bri102 reverse primer for amplification of CfTPS4


<220>
<221>  primer_bind
<222>  (1)..(25)
<223>  CfTPS4_R

<400>  18
gcttcacagc ctatgaatac atgat                                             25


<210>  19
<211>  22
<212>  DNA
<213>  Artificial sequence

<220>
<223>  Bri051 forward primer for amplification of CfTPS14


<220>
<221>  primer_bind
<222>  (1)..(22)
<223>  CfTPS14_F

<400>  19
tatgacacgg catgggttgc ta                                                22


<210>  20
<211>  29
<212>  DNA
<213>  Artificial sequence

<220>
<223>  Bri052 reverse primer for amplification of CfTPS14


<220>
<221>  primer_bind
<222>  (1)..(29)
<223>  CfTPS14_R

<400>  20
tcactcaaaa tttattctaa gacaagagg                                         29


<210>  21
<211>  26
<212>  DNA
<213>  Artificial sequence

<220>
<223>  Bri064 forward primer for E. coli expression of CfTPS1


<220>
<221>  primer_bind
<222>  (1)..(26)
<223>  CfTPS1_PciI_pmF

<400>  21
gctttagcaa catgtcatgg atgaac                                            26


<210>  22
<211>  22
<212>  DNA
<213>  Artificial sequence

<220>
<223>  Bri065 reverse primer for E. coli expression of CfTPS1


<220>
<221>  primer_bind
<222>  (1)..(22)
<223>  CfTPS1_XhoI_R

<400>  22
cagcactcga gggcgactgg tt                                                22


<210>  23
<211>  25
<212>  DNA
<213>  Artificial sequence

<220>
<223>  Bri068 forward primer for E. coli expression of CfTPS2


<220>
<221>  primer_bind
<222>  (1)..(25)
<223>  CfTPS2_BspHI_pmF

<400>  23
cacaagtaat catgagtcga gttgc                                          25


<210>  24
<211>  23
<212>  DNA
<213>  Artificial sequence

<220>
<223>  Bri069 reverse primer for E. coli expression of CfTPS2


<220>
<221>  primer_bind
<222>  (1)..(23)
<223>  CfTPS2_XhoI_R

<400>  24
ccaatgttct cgagcactgg ttc                                            23


<210>  25
<211>  37
<212>  DNA
<213>  Artificial sequence

<220>
<223>  Bri146 forward primer for E. coli expression of CfTPS3


<220>
<221>  primer_bind
<222>  (1)..(37)
<223>  CfTPS3_NcoI_inFusion_F

<400>  25
aggagatata ccatggctcc gatgatcacc tctaaat                             37


<210>  26
<211>  35
<212>  DNA
<213>  Artificial sequence

<220>
<223>  Bri147 reverse primer for E. coli expression  of CfTPS3


<220>
<221>  primer_bind
<222>  (1)..(35)
<223>  CfTPS3_XhoI_inFusion_R

<400>  26
ggtggtggtg ctcgaggttg ctgacacaac tcatt                               35


<210>  27
<211>  26
<212>  DNA
<213>  Artificial sequence

<220>
<223>  Bri117 forward primer for E. coli expression of CfTPS4


<220>
<221>  primer_bind
<222>  (1)..(26)
<223>  CfTPS4_BspHI_pmF

<400>  27
catcctttgt catgaaatgc agcctc                                          26


<210>  28
<211>  29
<212>  DNA
<213>  Artificial sequence

<220>
<223>  Bri118 reverse primer for E. coli expression of CfTPS4


<220>
<221>  primer_bind
<222>  (1)..(29)
<223>  CfTPS4_NotI_R

<400>  28
ttgttaggcg gccgctggag ggtgaattt                                       29


<210>  29
<211>  25
<212>  DNA
<213>  Artificial sequence

<220>
<223>  Bri066 forward primer for E. coli expression of CfTPS14


<220>
<221>  primer_bind
<222>  (1)..(25)
<223>  CfTPS14_NcoI_pmF

<400>  29
ttacgccatg gcttccctgg aagtt                                           25


<210>  30
<211>  27
<212>  DNA
<213>  Artificial sequence

<220>
<223>  Bri 067 reverse primer for E. coli expression of CfTPS14


<220>
<221>  primer_bind
<222>  (1)..(27)
<223>  CfTPS14_XhoI_R

<400>  30
actcaactcg agttctaaga caagagg                                          27


<210>  31
<211>  33
<212>  DNA
<213>  Artificial sequence

<220>
<223>  oSSB156 forward primer for tobacco expression of CfTPS1


<220>
<221>  primer_bind
<222>  (1)..(33)
<223>  CfTPS1 (f)

<400>  31
ggcttaauat ggggtctcta tccactatga acc                                   33


<210>  32
<211>  26
<212>  DNA
<213>  Artificial sequence

<220>
<223>  oSSB157 reverse primer for tobacco expression of CfTPS1


<220>
<221>  primer_bind
<222>  (1)..(26)
<223>  CfTPS1 (rc)

<400>  32
ggtttaautc aggcgactgg ttcgaa                                           26


<210>  33
<211>  34
<212>  DNA
<213>  Artificial sequence

<220>
<223>  oSSB158 forward primer for tobacco expression of CfTPS2


<220>
<221>  primer_bind
<222>  (1)..(34)
<223>  CfTPS2 (f)

<400>  33
ggcttaauat gaaaatgttg atgatcaaaa gtca                                  34


<210>  34
<211>  35
<212>  DNA
<213>  Artificial sequence

<220>
<223>  oSSB159 reverse primer for tobacco expression of CfTPS2


<220>
<221>  primer_bind
<222>  (1)..(35)
<223>  CfTPS2 (rc)

<400>  34
ggtttaautc agaccactgg ttcaaatagt acttg                                35


<210>  35
<211>  24
<212>  DNA
<213>  Artificial sequence

<220>
<223>  oSSB178 forward primer for tobacco expression of CfTPS3


<220>
<221>  primer_bind
<222>  (1)..(24)
<223>  CfTPS3 (f)

<400>  35
ggcttaauat gtcgtccctc gccg                                            24


<210>  36
<211>  35
<212>  DNA
<213>  Artificial sequence

<220>
<223>  oSSB179 reverse primer for tobacco expression of CfTPS3


<220>
<221>  primer_bind
<222>  (1)..(35)
<223>  CfTPS3 (rc)

<400>  36
ggtttaauct agttgctgac acaactcatt ttttc                                35


<210>  37
<211>  31
<212>  DNA
<213>  Artificial sequence

<220>
<223>  oSSB180 forward primer for tobacco expression of CfTPS4


<220>
<221>  primer_bind
<222>  (1)..(31)
<223>  CfTPS4 (f)

<400>  37
ggcttaauat gtcaatcacc atcaaccttc g                                    31


<210>  38
<211>  32
<212>  DNA
<213>  Artificial sequence

<220>
<223>  oSSB181 reverse primer for tobacco expression of CfTPS4


<220>
<221>  primer_bind
<222>  (1)..(32)
<223>  CfTPS4 (rc)

<400>  38
ggtttaautt agttagcagg tggagggtga at                                   32


<210>  39
<211>  31
<212>  DNA
<213>  Artificial sequence

<220>
<223>  oSSB160 forward primer for tobacco expression of CfTPS14


<220>
<221>  primer_bind
<222>  (1)..(31)
<223>  CfTPS14 (f)

<400>  39
ggcttaauat gtctctcccg ctctctactt g                                    31


<210>  40
<211>  41
<212>  DNA
<213>  Artificial sequence

<220>
<223>  oSSB161 reverse primer for tobacco expression of CfTPS14


<220>
<221>  primer_bind
<222>  (1)..(41)
<223>  CfTPS14 (rc)

<400>  40
ggtttaautt attctaagac aagaggttga taaataattg c                         41


<210>  41
<211>  22
<212>  DNA
<213>  Artificial sequence

<220>
<223>  CfTPS1 forward primer for qPCR


<220>
<221>  primer_bind
<222>  (1)..(22)
<223>  CfTPS1.for

<400>  41
tagtctggaa gggctggaga at                                                22


<210>  42
<211>  24
<212>  DNA
<213>  Artificial sequence

<220>
<223>  CfTPS1 reverse primer for qPCR


<220>
<221>  primer_bind
<222>  (1)..(24)
<223>  CfTPS1.rev

<400>  42
ttggtagcat ttaggatcac gagt                                              24


<210>  43
<211>  28
<212>  DNA
<213>  Artificial sequence

<220>
<223>  CfTPS2 forward primer for qPCR


<220>
<221>  primer_bind
<222>  (1)..(28)
<223>  CfTPS2.for

<400>  43
gacatattat cgcactatga atacaccg                                          28


<210>  44
<211>  29
<212>  DNA
<213>  Artificial sequence

<220>
<223>  CfTPS2 reverse primer for qPCR


<220>
<221>  primer_bind
<222>  (1)..(29)
<223>  CfTPS2.rev

<400>  44
ctaacttcac caatgtttgt atttcgagc                                          29


<210>  45
<211>  29
<212>  DNA
<213>  Artificial sequence

<220>
<223>  CfTPS3 forward primer for qPCR


<220>
<221>  primer_bind
<222>  (1)..(29)
<223>  CfTPS3.for

<400>  45
catgcagaat tggttgttat ttgtactca                                          29


<210>  46
<211>  27
<212>  DNA
<213>  Artificial sequence

<220>
<223>  CfTPS3 reverse primer for qPCR


<220>
<221>  primer_bind
<222>  (1)..(27)
<223>  CfTPS3.rev

<400>  46
tttctccact tgcattatta gcttcag                                            27


<210>  47
<211>  25
<212>  DNA
<213>  Artificial sequence

<220>
<223>  CfTPS4 forward primer for qPCR


<220>
<221>  primer_bind
<222>  (1)..(25)
<223>  CfTPS4.for

<400>  47
aacggaaaga aaatacagga aacgc                                              25


<210>  48
<211>  26
<212>  DNA
<213>  Artificial sequence

<220>
<223>  CfTPS4 reverse primer for qPCR


<220>
<221>  primer_bind
<222>  (1)..(26)
<223>  CfTPS4.rev

<400>  48
cagccaaata tttcgctttt gctatg                                          26


<210>  49
<211>  27
<212>  DNA
<213>  Artificial sequence

<220>
<223>  CfTPS14 forward primer for qPCR


<220>
<221>  primer_bind
<222>  (1)..(27)
<223>  CfTPS14 (f)

<400>  49
atgcatatgt atcatttgct ctagggc                                         27


<210>  50
<211>  30
<212>  DNA
<213>  Artificial sequence

<220>
<223>  CfTPS14 reverse primer for qPCR


<220>
<221>  primer_bind
<222>  (1)..(30)
<223>  CfTPS14 .rev

<400>  50
ggatttgaat agattgtggt agtcagcatg                                      30


<210>  51
<211>  20
<212>  DNA
<213>  Artificial sequence

<220>
<223>  CfEF1a forward primer for qPCR reference


<220>
<221>  primer_bind
<222>  (1)..(20)
<223>  CfEF1a (f)

<400>  51
tgcatcacga ggctctccag                                                   20


<210>  52
<211>  20
<212>  DNA
<213>  Artificial sequence

<220>
<223>  CfEF1a reverse primer for qPCR reference


<220>
<221>  primer_bind
<222>  (1)..(20)
<223>  CfEF1a (rc)

<400>  52
ggcaacaaac ccacgcttca                                                   20


<210>  53
<211>  23
<212>  DNA
<213>  Artificial sequence

<220>
<223>  CfH3 forward primer for qPCR reference


<220>
<221>  primer_bind
<222>  (1)..(23)
<223>  CfH3 (f)

<400>  53
gagattcgca agtaccagaa gag                                               23


<210>  54
<211>  23
<212>  DNA
<213>  Artificial sequence

<220>
<223>  CfH3 reverse primer for qPCR reference


<220>
<221>  primer_bind
<222>  (1)..(23)
<223>  CfH3 (rc)

<400>  54
aatcgcagat cagtcttgaa gtc                                               23


<210>  55
<211>  21
<212>  DNA
<213>  Artificial sequence

<220>
<223>  CfTIF4a forward primer for qPCR reference


<220>
<221>  primer_bind
<222>  (1)..(21)
<223>  CfTIF4a (f)

<400>  55
ctatgatctg ccaactcagc c                                                21


<210>  56
<211>  21
<212>  DNA
<213>  Artificial sequence

<220>
<223>  CfTIF4a reverse primer for qPCR reference


<220>
<221>  primer_bind
<222>  (1)..(21)
<223>  CfTIF4a (rc)

<400>  56
ccttggtcac gaagtttatg g                                                21


<210>  57
<211>  23
<212>  DNA
<213>  Artificial sequence

<220>
<223>  gCfTIF4a forward primer for qPCR gDNA check


<220>
<221>  primer_bind
<222>  (1)..(23)
<223>  gCfTIF4a (f)

<400>  57
tttgacatgy tgagaaggca gtc                                              23


<210>  58
<211>  21
<212>  DNA
<213>  Artificial sequence

<220>
<223>  gCfTIF4a reverse primer for qPCR gDNA check


<220>
<221>  primer_bind
<222>  (1)..(21)
<223>  gCfTIF4a (rc)

<400>  58
aacatagaac tgcttgatac c                                                 21


<210>  59
<211>  23
<212>  DNA
<213>  Artificial sequence

<220>
<223>  gCfEF1a forward primer for qPCR gDNA check


<220>
<221>  primer_bind
<222>  (1)..(23)
<223>  gCfEF1a (f)

<400>  59
tactactgca ctgtcattga tgc                                               23


<210>  60
<211>  22
<212>  DNA
<213>  Artificial sequence

<220>
<223>  gCfEF1a reverse primer for qPCR gDNA check


<220>
<221>  primer_bind
<222>  (1)..(22)
<223>  gCfEF1a (rc)

<400>  60
tggacctctc aatcatgttg tc                                                22


<210>  61
<211>  1710
<212>  DNA
<213>  Unknown

<220>
<223>  CfTPS3 codon optimised for E. coli


<220>
<221>  CfTPS3_co
<222>  (1)..(1710)
<223>  CfTPS3 from Coleus forskohlii, gene optimised

<400>  61
atgggcatta ccagcaagtc ctccgcagca gtcaaatgta gcctgaccac gccgaccgac       60

ctgatgggca agattaaaga agttttcaat cgtgaagtgg ataccagccc ggcagcaatg      120

accacgcaca gtacggacat cccgtccaac ctgtgcatta tcgataccct gcaacgtctg      180

ggcatcgacc aatattttca gtctgaaatt gatgcagttc tgcatgacac ctaccgcctg      240

tggcagctga aaaagaaaga tatctttagt gacatcacca cgcacgcgat ggccttccgt 300

ctgctgcgtg tcaaaggtta tgaagtggcg agcgatgaac tggcaccgta cgctgaccaa 360

gaacgtatca atctgcagac cattgatgtc ccgacggtgg ttgaactgta tcgtgcagct 420

caggaacgcc tgaccgaaga agatagtacg ctggaaaagc tgtacgtttg gaccagcgcg 480

tttctgaaac agcaactgct gacggatgcc attccggaca agaaactgca taagcaggtt 540

gaatattacc tgaaaaacta tcacggcatc ctggatcgca tgggtgtccg tcgcaatctg 600

gatctgtatg acattagcca ttacaagtct ctgaaagcgg cccaccgttt ctacaacctg 660

tcaaatgaag atatcctggc atttgctcgc caagacttca acatttcgca ggcacaacat 720

cagaaagaac tgcagcaact gcagcgttgg tatgctgatt gtcgcctgga caccctgaaa 780

tttggccgtg atgtcgtgcg catcggtaat ttcctgacga gcgccatgat tggcgatccg 840

gaactgtctg acctgcgtct ggcgtttgcc aaacatatcg ttctggtcac ccgcattgat 900

gactttttcg atcacggcgg tccgaaggaa gaaagctatg aaatcctgga actggtgaaa 960

gaatggaagg aaaaaccggc gggcgaatat gtctctgaag aagtggaaat cctgtttacc 1020

gccgtttaca acacggtcaa tgaactggcg gaaatggccc acattgaaca gggtcgtagt 1080

gtgaaggatc tgctggtgaa actgtgggtt gaaatcctgt ccgttttccg cattgaactg 1140

gatacctgga cgaacgacac cgcactgacg ctggaagaat atctgagtca gtcctgggtg 1200

tcaattggct gccgcatttg tatcctgatt tcgatgcaat ttcagggtgt taaactgtca 1260

gatgaaatgc tgcagtcgga agaatgcacc gacctgtgtc gttacgtgag catggttgat 1320

cgcctgctga atgacgtcca aaccttcgaa aaggaacgta aagaaaacac gggcaattca 1380

gtgtcgctgc tgcaggcagc tcataaagat gaacgcgtta tcaacgaaga agaagcgtgc 1440

attaaggtca aagaactggc tgaatataat cgtcgcaagc tgatgcagat cgtgtacaaa 1500

accggtacga tttttccgcg taagtgtaaa gacctgtttc tgaaagcgtg ccgcattggc 1560

tgttatctgt acagctctgg tgatgaattt accagcccgc agcaaatgat ggaagacatg 1620

aaatctctgg tttatgaacc gctgccgatt agcccgccgg aagccaacaa tgcgtcaggt 1680

gaaaagatgt cgtgtgtctc aaacctcgag 1710